(19)

Europäisches
Patentamt
European
Patent Office
Office européen
des brevets

(11)  **EP 4 174 170 A1**

(12)  **EUROPEAN PATENT APPLICATION**
published in accordance with Art. 153(4) EPC

(43) Date of publication:
**03.05.2023  Bulletin 2023/18**

(21) Application number: **21828039.4**

(22) Date of filing: **24.06.2021**

(51) International Patent Classification (IPC):
*C12N 5/071* (2010.01)    *A61K 35/38* (2015.01)
*A61L 27/38* (2006.01)    *A61P 1/00* (2006.01)
*C12Q 1/02* (2006.01)    *G01N 33/15* (2006.01)
*G01N 33/50* (2006.01)    *C12N 5/10* (2006.01)

(52) Cooperative Patent Classification (CPC):
**A61K 35/38; A61L 27/38; A61P 1/00; C12N 5/06;
C12Q 1/02; G01N 33/15; G01N 33/50;** C12N 5/10

(86) International application number:
**PCT/JP2021/023893**

(87) International publication number:
**WO 2021/261540 (30.12.2021 Gazette 2021/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **25.06.2020  JP 2020109311
08.12.2020  JP 2020203679**

(71) Applicant: **FUJIFILM Corporation
Tokyo 106-8620 (JP)**

(72) Inventors:
• **IMAKURA Yuki
Ashigarakami-gun, Kanagawa 258-8577 (JP)**
• **MIMA Shinji
Ashigarakami-gun, Kanagawa 258-8577 (JP)**
• **OGURA Izumi
Ashigarakami-gun, Kanagawa 258-8577 (JP)**
• **YAMAZAKI Nao
Ashigarakami-gun, Kanagawa 258-8577 (JP)**

(74) Representative: **Hoffmann Eitle
Patent- und Rechtsanwälte PartmbB
Arabellastraße 30
81925 München (DE)**

(54)  **PRODUCTION METHOD FOR INTESTINAL EPITHELIAL CELLS AND USE THEREFOR**

(57)  An object of the present invention is to provide a method of producing an intestinal epithelial cell, which has a large number of cells per area and a high accuracy of kinetic prediction for a CYP3A4 substrate drug such as midazolam, by inducing the differentiation of a pluripotent stem cell, as well as the intestinal epithelial cell, a cell sheet, an evaluation method for a test substance, a screening kit for a test substance, and a cell preparation. According to the present invention, there is provided a production method for an intestinal epithelial cell, including a first differentiation step of differentiating a pluripotent stem cell into an intestinal stem cell, a proliferation step of proliferating the intestinal stem cell obtained in the differentiation step, and a second differentiation step of differentiating the intestinal stem cell obtained in the proliferation step into an intestinal epithelial cell, in which the proliferation step is a step of bringing the intestinal stem cell into a specific state.

EP 4 174 170 A1

**Description**

Field of the Invention

The present invention relates to a production method for an intestinal epithelial cell, which includes inducing the differentiation of a pluripotent stem cell. The present invention further relates to an intestinal epithelial cell, a cell sheet, an evaluation method for a test substance, a screening kit for a test substance, and a cell preparation.

Description of the Related Art

Since many drug metabolizing enzymes and drug transporters are present in small intestine, the small intestine is very important as an organ involved in the first-pass effect of drugs, like the liver. As a result, for the development of a pharmaceutical drug having excellent pharmacokinetic properties, it is necessary to evaluate the membrane permeability and metabolism of the pharmaceutical drug in the small intestine from the early stage of pharmaceutical drug development. At present, Caco-2 cells derived from human colon cancer are frequently used as a small intestine model system. However, the expression pattern of a drug transporter in Caco-2 cells is different from that in the human small intestine. In addition, it is difficult to accurately evaluate the pharmacokinetics in the small intestine, since Caco-2 cells hardly exhibit the expression and induction of a drug metabolizing enzyme. Accordingly, it is desirable to use primary small intestine epithelial cells in order to comprehensively evaluate drug metabolism and membrane permeability in the small intestine, but it is difficult to widely use the primary intestinal epithelial cells as a pharmacokinetic test system like primary hepatocytes due to problems in terms of function and supply.

Human induced pluripotent stem (iPS) cells were established in 2007 by Yamanaka et al. The human iPS cells are cells having multiple differentiation potency and almost infinite proliferation ability that are similar to those of human embryonic stem (ES) cells established by Thomson et al. in 1998. The human iPS cells have few ethical problems as compared with the human ES cells and are expected to be a stable cell source for pharmaceutical drug development.

As a culture method that enables the maintenance and culture of an induced pluripotent stem cell-derived intestinal stem cell while maintaining the properties of the intestinal stem cell, Patent Document 1 discloses culturing an induced pluripotent stem cell-derived intestinal stem cell-like cell in the presence of a GSK-3β inhibitor, a histone deacetylation inhibitor, and a serum substitute, or in the presence of a GSK-3β inhibitor and a serum substitute.

Patent Document 2 discloses a method of inducing the differentiation of a pluripotent stem cell into an intestinal epithelial cell, which includes (1) a step of differentiating a pluripotent stem cell into an intestinal stem cell-like cell; and (2) using in combination a MEK1 inhibitor, a DNA methylation inhibitor, a TGFβ receptor inhibitor, EGF, and a cAMP activator to differentiate the intestinal stem cell-like cell obtained in the step (1) into an intestinal epithelial cell-like cell.

Prior Art Documents

Patent Documents

Patent Document 1: WO2018/151307A
Patent Document 2: WO2019/156200A

**SUMMARY OF THE INVENTION**

The intestinal epithelial cell induced to be differentiated according to the method described in Patent Document 2 has a drug metabolizing enzyme (CYP3A4) activity comparable to that of the human primary small intestinal cell (an intestinal epithelial cell derived from a living body). On the other hand, there is a demand for the development of such a production method for an intestinal epithelial cell that can further increase the number of cells per area and further improve the accuracy of the kinetic prediction for a CYP3A4 substrate drug such as midazolam.

An object to be achieved in the present invention is to provide a method of producing an intestinal epithelial cell, which has a large number of cells per area and a high accuracy of kinetic prediction for a CYP3A4 substrate drug such as midazolam, by inducing the differentiation of a pluripotent stem cell. In addition, an object to be achieved in the present invention is to provide an intestinal epithelial cell, which has a large number of cells per area and a high accuracy of kinetic prediction for a CYP3A4 substrate drug such as midazolam, and a cell sheet containing the intestinal epithelial cell. Further, an object to be achieved in the present invention is to provide an evaluation method for a test substance, a screening kit for a test substance, and a cell preparation, in which the above-described intestinal epithelial cell or cell sheet is used.

The inventors of the present invention diligently studied in order to achieve the above-described objects. As a result, it was found that in a proliferation step in a production method for an intestinal epithelial cell, the production method including a first differentiation step of differentiating a pluripotent stem cell into an intestinal stem cell, a proliferation step of proliferating the intestinal stem cell obtained in the differentiation step, and a second differentiation step of differentiating the intestinal stem cell obtained in the proliferation step into an intestinal epithelial cell, in a case where the intestinal stem cell is brought into a specific state and then differentiated into an intestinal epithelial cell, the morphology and the number of cells of the intestinal epithelial cell after differentiation can be improved, and the accuracy of the kinetic prediction for midazolam can be improved. The present invention has been completed based on the above findings.

That is, according to the present invention, the following inventions are provided.

<1> A production method for an intestinal epithelial cell, comprising:

a first differentiation step of differentiating a pluripotent stem cell into an intestinal stem cell;
a proliferation step of proliferating the intestinal stem cell obtained in the differentiation step; and
a second differentiation step of differentiating the intestinal stem cell obtained in the proliferation step into an intestinal epithelial cell,
in which the proliferation step is any one of;
a step in which a density of the intestinal stem cells obtained in the first differentiation step becomes $30 \times 10^4$ cells/cm$^2$ or more,
a step in which the density of the intestinal stem cells obtained in the first differentiation step becomes 3 times or more as compared with a density of the intestinal stem cells immediately after the first differentiation step,
a step in which an EdU-positive proportion of the intestinal stem cells obtained in the first differentiation step becomes 90% or more, or
a step in which the EdU-positive proportion of the intestinal stem cells obtained in the first differentiation step becomes 2.0 times or more as compared with a proportion of the intestinal stem cells immediately after the first differentiation step.

<2> The production method for an intestinal epithelial cell according to <1>, in which the proliferation step includes a step of culturing the intestinal stem cell in a presence of a Wnt agonist or a GSK-3β inhibitor.

<3> The production method for an intestinal epithelial cell according to <1>, in which the proliferation step includes a step of culturing the intestinal stem cell in a presence of a GSK-3β inhibitor and a serum substitute.

<4> The production method for an intestinal epithelial cell according to any one of <1> to <3>, in which a cell density at an end of the first differentiation step is $5.0 \times 10^4$ cells/cm$^2$ to $20 \times 10^4$ cells/cm$^2$.

<5> The production method for an intestinal epithelial cell according to any one of <1> to <4>, in which in the proliferation step, the intestinal stem cells obtained in the first differentiation step are seeded in a container to a cell density of 1.1 to 3.3 times, and then the intestinal stem cells are cultured to be proliferated.

<6> The production method for an intestinal epithelial cell according to any one of <1> to <5>, in which the proliferation step is carried out for 1 to 10 days.

<7> The production method for an intestinal epithelial cell according to any one of <1> to <6>, in which the proliferation step and the second differentiation step are carried out in a culture container coated with an extracellular matrix of 3% by volume to 100% by volume.

<8> The production method for an intestinal epithelial cell according to any one of <1> to <6>, in which the proliferation step and the second differentiation step are carried out in a culture medium to which an extracellular matrix of 1% to 10% is added.

<9> The production method for an intestinal epithelial cell according to <8>, in which the proliferation step and the second differentiation step are carried out in a culture medium in which the extracellular matrix of 1% to 10% is added to only a lower bottom (basolateral) side a cell culture insert.

<10> The production method for an intestinal epithelial cell according to any one of <1> to <9>, in which the second differentiation step includes a step of differentiating the intestinal stem cell into the intestinal epithelial cell in a presence of a MEK1 inhibitor, a DNA methylation inhibitor, a TGFβ receptor inhibitor, EGF, and a cAMP activator.

<11> An intestinal epithelial cell that is produced by the method according to any one of <1> to <10>.

<12> A cell sheet comprising the intestinal epithelial cell according to <11>.

<13> A cell sheet comprising:
an intestinal epithelial cell derived from a pluripotent stem cell, in which a proportion of columnar intestinal epithelial cells in the intestinal epithelial cells is 10% or more.

<14> The cell sheet according to <13>, in which a cell density is $50 \times 10^4$ cells/cm$^2$ or more.

<15> The cell sheet according to <13> or <14>, in which an amount of a midazolam metabolite after incubation in a culture medium containing 5 μmol/L midazolam at 37°C for 2 hours is 300 pmol/cm$^2$ or more per area of the cell sheet.

<16> The cell sheet according to any one of <13> to <15>, in which a CYP3A4 activity per protein mass is 400 to 2,500 pmol/2 hr/mg·protein.

<17> The cell sheet according to any one of <13> to <16>, in which an apparent permeability coefficient of midazolam is $40 \times 10^{-6}$ cm/s or less.

<18> A evaluation method for a test substance, comprising bringing a test substance into contact with the intestinal epithelial cell according to <11> or the cell sheet according to any one of <12> to <17>.

<19> The evaluation method for a test substance according to <18>, further comprising correcting an apparent permeability coefficient using a scaling factor.

<20> The evaluation method for a test substance according to <18>, further comprising correcting Fg using a scaling factor.

<21> The evaluation method for a test substance according to <19> or <20>, in which the scaling factor is 0.1 to 500.

<22> A screening kit for a test substance, comprising the intestinal epithelial cell according to <11> or the cell sheet according to any one of <12> to <17>.

<23> A cell preparation comprising the intestinal epithelial cell according to <11> or the cell sheet according to any one of <12> to <17>.

According to the present invention, it is possible to provide an intestinal epithelial cell, which has a large number of cells per area and a high accuracy of kinetic prediction for a CYP3A4 substrate drug such as midazolam. According to the intestinal epithelial cell, the cell sheet, the evaluation method of a test substance, and the screening kit for a test substance according to aspects of the present invention, it is possible to predict the kinetics of a test substance with high accuracy.

## BRIEF DESCRIPTION OF THE DRAWINGS

Fig. 1 is a diagram illustrating an outline of the differentiation culture methods of Comparative Example 1 and Examples 1 to 5.
Fig. 2 is a section image showing intestinal epithelial cells of Comparative Example 1.
Fig. 3 is a section image showing intestinal epithelial cells of Example 1.
Fig. 4 is a section image showing intestinal epithelial cells of Example 3.
Fig. 5 is a section image showing intestinal epithelial cells of Example 4.
Fig. 6 is a section image showing intestinal epithelial cells of Example 5.

## DESCRIPTION OF THE PREFERRED EMBODIMENTS

Hereinafter, embodiments of the present invention will be described below.

The terms in the present specification have the following meanings.

5-aza-2'dc: 5-aza-2'-deoxycytidine
bFGF: Basic Fibroblast Growth Factor
BMP4: Bone morphogenetic protein 4
cAMP: Cyclic adenosine monophosphate
Cript: Cysteine-rich PDZ-binding protein
DAPI: 4',6-diamidino-2-phenylindole
DMEM: Dulbecco's Modified Eagle's culture Medium
D-PBS: Dulbecco's Phosphate-Buffered Saline
EdU: 5-ethynyl-2'-deoxyuridine
EGF: Epidermal Growth Factor
FBS: Fetal Bovine Serum
Fbxo15: F-Box Protein 15
FGF2: Fibroblast Growth Factor-2
GSK-3$\beta$: Glycogen synthase kinase 3$\beta$
HBSS: Hank's Balanced Salt Solution
LIF: Leukemia inhibitory factor
MEK1: MAPK-ERK kinase 1
NEAA: Non-Essential Amino Acids

PKA: protein kinase A
SCF: Stem cell factor
TGFβ: Transforming Growth Factor-β
VEGF: vascular endothelial growth factor
Wnt: Wingless-type MMTV integration site family

<Production method for intestinal epithelial cell>

The production method for an intestinal epithelial cell according to the embodiment of the present invention is a method including:

a first differentiation step of differentiating a pluripotent stem cell into an intestinal stem cell;
a proliferation step of proliferating the intestinal stem cell obtained in the differentiation step; and
a second differentiation step of differentiating the intestinal stem cell obtained in the proliferation step into an intestinal epithelial cell,
in which the proliferation step is any one of;
a step in which a density of the intestinal stem cells obtained in the first differentiation step becomes $30 \times 10^4$ cells/cm$^2$ or more,
a step in which the density of the intestinal stem cells obtained in the first differentiation step becomes 2.0 times or more as compared with a density of the intestinal stem cells immediately after the first differentiation step,
a step in which an EdU-positive proportion of the intestinal stem cells obtained in the first differentiation step becomes 90% or more, or
a step in which the EdU-positive proportion of the intestinal stem cells obtained in the first differentiation step becomes 2.0 times or more as compared with a proportion of the intestinal stem cells immediately after the first differentiation step.

The method according to the embodiment of the present invention is characterized in that the density of intestinal stem cells is made to be a high density of a certain level or higher. In a case where a Wnt agonist or a GSK-3β inhibitor is added to the intestinal stem cells to increase the density thereof, the improvement in morphology and function of the intestinal epithelial cells is observed. On the other hand, in a case where the cell density is insufficient, the cell morphology and the kinetics of the substrate of the metabolic enzyme are not improved. There has been no finding that pluripotent stem cells derived from the pluripotent stem cell differentiated on a plane can be made to have a morphology of a column shape close to that of a living body by causing the density of the intestinal stem cells to be a high density of a certain level or higher, and thus the present invention has an advantageous effect.

In the present invention, a pluripotent stem cell is induced to be differentiated into an intestinal epithelial cell lineage. According to the present invention, cells that exhibit characteristics similar to intestinal epithelial cells which constitute the intestinal tissue of the living body, that is, intestinal epithelial cells are obtained.

"Pluripotent stem cells" refer to cells having the ability (differentiation pluripotency) to differentiate into all cells that constitute a living body and the ability (the self-replication ability) to generate daughter cells having the same differentiation potency as that of the pluripotent stem cells through cell division. Differentiation pluripotency can be evaluated by transplanting cells to be evaluated into nude mice and testing for the presence or absence of formation of teratoma including cells of each of the three germ layers (ectoderm, mesoderm, and endoderm).

Examples of the pluripotent stem cell include an embryonic stem cell (an ES cell), an embryonic germ cell (an EG cell), and an induced pluripotent stem cell (an iPS cell); however, examples thereof are not limited thereto as long as a cell has both differentiation pluripotency and self-replication ability. An ES cell or an iPS cell is preferably used. An iPS cell is more preferably used. The pluripotent stem cell is preferably a mammalian (for example, a primate such as a human or a chimpanzee or a rodent such as a mouse or a rat) cell and particularly preferably a human cell. Accordingly, in the most preferred embodiment of the present invention, a human iPS cell is used as the pluripotent stem cell.

The ES cell can be established, for example, by culturing an early embryo before implantation, an inner cell mass constituting the early embryo, a single blastomere, or the like (Manipulating the Mouse Embryo A Laboratory Manual, Second Edition, Cold Spring Harbor Laboratory Press (1994); Thomson, J. A. et. al., Science, 282, 1145-1147 (1998)). As the early embryo, an early embryo produced by nuclear transplantation of a nucleus of a somatic cell may be used (Wilmut et al. (Nature, 385, 810 (1997)), Cibelli et al. (Science, 280, 1256 (1998)), Iriya et al (Protein Nucleic Acid Enzyme, 44, 892 (1999)), Baguisi et al. (Nature Biotechnology, 17, 456 (1999)), Wakayama et al. (Nature, 394, 369 (1998); Nature Genetics, 22, 127 (1999); Proc. Natl. Acad. Sci. USA, 96, 14984 (1999)), Rideout III et al. (Nature Genetics, 24, 109 (2000)), Tachibana et al. (Human Embryonic Stem Cells Derived by Somatic Cell Nuclear Transfer, Cell (2013) in press)). As the early embryo, a parthenogenetic embryo may be used (Kim et al. (Science, 315, 482-486 (2007)), Nakajima et al. (Stem Cells, 25, 983-985 (2007)), Kim et al. (Cell Stem Cell, 1, 346-352 (2007)), Revazova et al. (Cloning Stem Cells, 9, 432-449 (2007)), Revazova et al. (Cloning Stem Cells, 10, 11-24 (2008)). In addition to the above-described papers, the production of the ES cell is described in Strelchenko N., et al. Reprod Biomed Online. 9: 623-629, 2004; KlimanskayaI., et al. Nature 444: 481-485, 2006; Chung Y. et al., Cell Stem Cell 2: 113-117, 2008; Zhang X., et al. Stem Cells 24:2669-2676, 2006; Wassarman, P. M. et al. Methods in Enzymology, Vol. 365, 2003, or the like. In addition, fused ES cells obtained by cell fusion of ES cells with somatic cells are also included in the embryonic stem cells used in the method according to the embodiment of the present invention.

Some ES cells are available from conservation institutions or are commercially available. For example, human ES cells are available from the Institute for Frontier Medical Sciences, Kyoto University (for example, KhES-1, KhES-2, and KhES-3), WiCell Research Institute, ESI BIO.

An EG cell can be established by culturing a primordial germ cell in the presence of LIF, bFGF, and SCF (Matsui et al., Cell, 70, 841-847 (1992), Shamblott et al., Proc. Natl. Acad. Sci. USA, 9523, 13726-13731 (1998), Turnpenny et al., Stem Cells, 21 (5), 598-609, (2003)).

"Induced pluripotent stem cells (iPS cells)" are cells that have pluripotency (multiple differentiation potency) and proliferation ability and that are produced by reprogramming somatic cells by introducing reprogramming factors or the like. The induced pluripotent stem cells exhibit properties similar to the ES cells. The somatic cells used for producing iPS cells are not particularly limited and may be differentiated somatic cells or undifferentiated stem cells. In addition, the origin of the somatic cells is not particularly limited but preferably somatic cells of mammals (for example, primates such as a human and a chimpanzee, rodents such as a mouse and a rat) and particularly preferably human somatic cells. For example, a human adult-derived somatic cell (that is, a mature somatic cell) may be used in addition to the fetal somatic cell. Examples of the somatic cell include: (1) tissue stem cells (somatic stem cells) such as a neural stem cell, a hematopoietic stem cell, a mesenchymal stem cell, and a dental pulp stem cell; (2) tissue progenitor cells; and (3) differentiated cells such as a fibroblast (a skin cell and the like), an epithelial cell, a hepatocyte, a lymphocyte (T cell or B cell), an endothelial cell, a muscle cell, a hair cell, a gastric mucosal cell, an intestinal cell, a splenocyte, a pancreatic cell (an exocrine pancreatic cell and the like), a brain cell, a lung cell, a kidney cell, and a skin cell. The iPS cells can be produced by various methods reported so far. In addition, it is naturally expected that an iPS cell production method to be developed in the future will be applied.

The most basic producing method for an iPS cell is a method in which four transcription factors, Oct3/4, Sox2, Klf4, and c-Myc are introduced into a cell using a virus (Takahashi K, Yamanaka S: Cell 126 (4), 663-676, 2006; Takahashi, K, et al.: Cell 131 (5), 861-72, 2007). It has been reported that human iPS cells have been established by introducing four factors, Oct4, Sox2, Lin28, and Nanog (Yu J, et al.: Science 318 (5858), 1917-1920, 2007). It has also been reported that iPS cells have been established by introducing three factors excluding c-Myc (Nakagawa M, et al.: Nat. Biotechnol. 26 (1), 101-106, 2008), two factors of Oct3/4 and Klf4 (Kim J B, et al.: Nature 454 (7204), 646-650, 2008), or Oct3/4 alone (Kim J B, et al.: Cell 136 (3), 411-419, 2009). In addition, a method for introducing a protein, which is an expression product of a gene, into cells (Zhou H, Wu S, Joo J Y, et al.: Cell Stem Cell 4, 381-384, 2009; Kim D, Kim C H, Moon J I, et al.: Cell Stem Cell 4, 472-476, 2009) has also been reported. On the other hand, it has also been reported that by using BIX-01294 which is an inhibitor of histone methyltransferase G9a, valproic acid (VPA) which is a histone deacetylase inhibitor, or Bay K8644, the production efficiency has been improved and the factors to be introduced have been reduced (Huangfu D, et al.: Nat. Biotechnol. 26 (7), 795-797, 2008; Huangfu D, et al.: Nat. Biotechnol. 26 (11), 1269-1275, 2008; Silva J, et al.: PLoS. Biol. 6 (10), e253, 2008). Studies on gene transfer methods have also been carried out, and technologies for gene transfer have been developed using, in addition to a retrovirus, a lentivirus (Yu J, et al.: Science 318 (5858), 1917-1920, 2007), an adenovirus (Stadtfeld M, et al.: Science 322 (5903), 945-949, 2008), a plasmid (Okita K, et al.: Science 322 (5903), 949-953, 2008), a transposon vector (Woltjen K, Michael I P, Mohseni P, et al.: Nature 458, 766-770, 2009; Kaji K, Norrby K, Paca A, et al.: Nature 458, et al. 771-775, 2009; Yusa K, Rad R, Takeda J, et al.: Nat Methods 6, 363-369, 2009) or an episomal vector (Yu J, Hu K, Smuga-Otto K, Tian S, et al.: Science 324, 797-801, 2009).

Cells transformed to iPS cells, that is, cells that have undergone initialization (reprogramming) can be selected using, as an indicator, the expression of pluripotent stem cell markers (undifferentiation markers) such as Fbxo15, Nanog, Oct3/4, Fgf-4, Esg-1, and Cript, or the like. The selected cells are recovered as iPS cells.

The iPS cells can be available from, for example, National University Corporation Kyoto University, or Independent Administrative Institution RIKEN BioResource Center.

In the present specification, "inducing differentiation" refers to acting to differentiate along a specific cell lineage. In the present invention, iPS cells are induced to be differentiated into intestinal epithelial cells.

The production method for an intestinal epithelial cell according to the present invention includes three stages of steps which are classified roughly (a first differentiation step of differentiating a pluripotent stem cell into an intestinal stem cell; a proliferation step of proliferating the intestinal stem cell obtained in the differentiation step; and a second differentiation step of differentiating the intestinal stem cell obtained in the proliferation step into an intestinal epithelial cell).

<First differentiation step: differentiation into intestinal stem cell>

In the first differentiation step, pluripotent stem cells are cultured and differentiated into intestinal stem cells. In other words, pluripotent stem cells are cultured under conditions that induce differentiation into intestinal stem cells. The culture conditions are not particularly limited as long as the pluripotent stem cells differentiate into intestinal stem cells. Typically, differentiation induction of two stages described below, that is, the differentiation of the pluripotent stem cells into endoderm-like cells (a step (1-1)) and the differentiation of endoderm-like cells into intestinal stem cells (a step (1-2)) are carried out so that the pluripotent stem cells differentiate into intestinal stem cells via endoderm-like cells.

Step (1-1) differentiation into endoderm-like cell

In this step, pluripotent stem cells are cultured and differentiated into endoderm-like cells. In other words, the pluripotent stem cells are cultured under conditions that induce differentiation into the endoderm-like cell. The culture conditions are not particularly limited as long as the pluripotent stem cells differentiate into endoderm-like cells. For example, the pluripotent stem cells are cultured in a culture medium to which activin A is added, according to a conventional method. In this case, the concentration of activin A in the culture medium is set to, for example, 10 ng/mL to 200 ng/mL and preferably 20 ng/mL to 150 ng/mL. It is preferable to add serum or a serum substitute (KnockOut™ Serum Replacement (KSR) or the like) to the culture medium from the viewpoints of the cell growth rate and cell maintenance rate. The serum is not limited to fetal bovine serum, and human serum, sheep serum, or the like can be also used. The addition amount of serum or a serum substitute is, for example, 0.1% (v/v) to 10% (v/v).

An inhibitor of the Wnt/$\beta$-catenin signaling pathway (for example, hexachlorophene, quercetin, or Wnt3a which is a Wnt ligand) may be added to the culture medium to promote differentiation into endoderm-like cells.

One or more of BMP4, VEGF, and FGF2 may be added to the culture medium to promote differentiation into endoderm-like cells.

This step can be also carried out by the method described in WO2014/165663A or a method based thereon.

In a preferred aspect, two stage culture is carried out as the step (1-1). First stage culture is carried out in a culture medium to which a relatively low concentration of serum (for example, 0.1% (v/v) to 1% (v/v)) is added, and subsequent second stage culture is carried out in a culture medium having a higher serum concentration than the first stage culturing (for example, a serum concentration of 1% (v/v) to 10% (v/v)). Employing the two stage culture in this manner is preferable since the proliferation of undifferentiated cells is suppressed by the first stage culture and differentiated cells are proliferated by the subsequent second stage culture.

The period (the culture period) of the step (1-1) is, for example, 1 day to 10 days and preferably 2 days to 7 days. In a case where the two stage culture is employed as the step (1-1), the culture period of first stage is, for example, 1 day to 7 days and preferably 2 days to 5 days, and the culture period of second stage is, for example, 1 day to 6 days and preferably 1 day to 4 days.

Step 1-2: differentiation into intestinal stem cell

In this step, the endoderm-like cells obtained in the step (1-1) are cultured and differentiated into intestinal stem cells. In other words, the endoderm-like cells are cultured under conditions that induce differentiation into intestinal stem cells. The culture conditions are not particularly limited as long as the endoderm-like cells differentiate into intestinal stem cells. The culture is preferably carried out in the presence of FGF2 (fibroblast growth factor 2) or in the presence of a GSK-3$\beta$ inhibitor. Human FGF2 (for example, a human recombinant FGF2) is preferably used as FGF2.

Typically, the cell population or a part thereof obtained through the step (1-1) is used in the step (1-2) without sorting. Alternatively, the step (1-2) may be carried out after sorting endoderm-like cells from the cell population obtained through step (1-1). The sorting of endoderm-like cells may be carried out, for example, with a flow cytometer (cell sorter) using a cell surface marker as an indicator.

"In the presence of FGF2" is synonymous with under the condition in which FGF2 is added to a culture medium. Accordingly, in order to carry out culture in the presence of FGF2, a culture medium to which FGF2 is added may be used. An example of the concentration of FGF2 to be added is 100 ng/mL to 500 ng/mL.

Similarly, "in the presence of a GSK-3$\beta$ inhibitor" is synonymous with under the conditions in which a GSK-3$\beta$ inhibitor has been added to a culture medium. Accordingly, in order to carry out culture in the presence of GSK-3$\beta$ inhibitor, a culture medium to which GSK-3$\beta$ inhibitor has been added may be used. Examples of the GSK-3$\beta$ inhibitor include CHIR99021, SB216763, CHIR98014, TWS119, Tideglusib, SB415286, BIO, AZD2858, AZD1080, AR-A014418, TDZD-8, LY2090314, IM-12, Indirubin, Bikinin, and 1-Azakenpaullone. The concentration of the GSK-3$\beta$ inhibitor to be added may be 0.1 nmol/L to 30 $\mu$mol/L, and it is preferably 1 nmol/L to 10 $\mu$mol/L, more more preferably 10 nmol/L to 10 $\mu$mol/L, and still more preferably 50 nmol/L to 5 $\mu$mol/L.

The period (the culture period) of the step (1-2) is, for example, 2 days to 10 days and preferably 3 days to 7 days. In a case where the culture period is too short, an expected effect (increase in differentiation efficiency or promotion of acquisition of functions as intestinal stem cells) cannot be sufficiently obtained. On the other hand, in a case where the culture period is too long, the differentiation efficiency will be reduced.

The differentiation into intestinal stem cells can be determined or evaluated using, for example, the expression of an intestinal stem cell marker as an indicator. Examples of the intestinal stem cell marker include G protein-coupled receptor 5 (LGR5) containing leucine-rich repeats and Ephrin B2 receptor (EphB2).

The cell density at the end of the first differentiation step may be $5.0 \times 10^4$ cells/cm$^2$ to $20 \times 10^4$ cells/cm$^2$, and it is preferably $6.0 \times 10^4$ cells/cm$^2$ to $16 \times 10^4$ cells/cm$^2$ and more preferably $7.0 \times 10^4$ cells/cm$^2$ to $14 \times 10^4$ cells/cm$^2$.

The intestinal stem cells obtained in the step (1-2) can also be cryopreserved. The cryopreserved intestinal stem cells may be thawed and a subsequent step (the proliferation step or the like) may be carried out. For the cryopreservation of the cells, a solution generally used for the cryopreservation of cultured cells can be used, and a culture medium, DMSO, serum, a general cryopreservation reagent, and the like may be contained. Examples of the general cryopreservation reagent include CryoStor (registered trade name) Freeze Media, CellBanker (registered trade name), FreSR (registered trade name)-S, NutriFreez (registered trade name) D10 Cryopreservation culture medium, Cellvation, Cryoscarless (registered trade name), and Bambanker (registered trade name).

<Proliferation step>

The proliferation step is any one of;

a step (hereinafter, referred to as a step a) in which a density of the intestinal stem cells obtained in the first differentiation step becomes $30 \times 10^4$ cells/cm$^2$ or more,
a step (hereinafter, referred to as a step b) in which the density of the intestinal stem cells obtained in the first differentiation step becomes 2.0 times or more as compared with a density of the intestinal stem cells immediately after the first differentiation step,
a step (hereinafter, referred to as a step c) in which an EdU-positive proportion of the intestinal stem cells obtained in the first differentiation step becomes 90% or more, or
a step (hereinafter, referred to as a step d) in which the EdU-positive proportion of the intestinal stem cells obtained in the first differentiation step becomes 3 times or more as compared with a proportion of the intestinal stem cells immediately after the first differentiation step.

In the step a, the density of the intestinal stem cells obtained in the first differentiation step becomes $30 \times 10^4$ cells/cm$^2$ or more, preferably becomes $32 \times 10^4$ cells/cm$^2$ or more, more preferably becomes $40 \times 10^4$ cells/cm$^2$ or more, and particularly preferably becomes $45 \times 10^4$ cells/cm$^2$ or more.

In the step b, the density of the intestinal stem cells obtained in the first differentiation step becomes 2.0 times or more, preferably becomes 2.5 times or more, more preferably 3.0 times or more, still more preferably becomes 3.5 times or more, and particularly preferably becomes 4.0 times or more, as compared with the density of the intestinal stem cells immediately after the first differentiation step.

In the step c, the EdU-positive proportion of the intestinal stem cells obtained in the first differentiation step becomes 90% or more, preferably becomes 95% or more, more preferably becomes 97% or more, and particularly preferably becomes 100%.

In the step d, the EdU-positive proportion of the intestinal stem cells obtained in the first differentiation step becomes 3 times or more, preferably becomes 3.2 times or more, and more preferably becomes 3.5 times or more, as compared with the proportion immediately after the first differentiation step.

In the proliferation step, the intestinal stem cells obtained in the first differentiation step can be cultured to be proliferated after seeding, in a container, the intestinal stem cells to a seeding density of preferably 1.1 to 3.3 times, more preferably 1.5 to 3.0 times, still more preferably 1.5 to 2.5 times, and particularly preferably 1.5 to 2.0 times with respect to the cell density of the intestinal stem cells obtained in the first differentiation step.

The culture conditions of the proliferation step are not particularly limited as long as the conditions of any one of the above-described steps a to d are satisfied. Preferably, the intestinal stem cells can be cultured in the presence of a Wnt agonist or a GSK-3β inhibitor.

Examples of the Wnt agonist include a Wnt family such as Wnt3a and an R-spondin family such as R-spondin1. The concentration of the Wnt agonist to be added is 10 ng/mL to 10 μg/mL, and it is preferably 100 ng/mL to 1 μg/mL and more preferably 100 ng/mL to 500 ng/mL.

Examples of the GSK-3β inhibitor include CHIR99021, SB216763, CHIR98014, TWS119, Tideglusib, SB415286, BIO, AZD2858, AZD1080, AR-A014418, TDZD-8, LY2090314, IM-12, Indirubin, Bikinin, and 1-Azakenpaullone. The concentration of the GSK-3β inhibitor to be added is 0.1 nmol/L to 30 μmol/L, and it is preferably 1 nmol/L to 10 μmol/L, more preferably 10 nmol/L to 10 μmol/L, still more preferably 50 nmol/L to 5.0 μmol/L, and particularly

preferably 1.0 µmol/L to 5.0 µmol/L. The concentration of the GSK-3β inhibitor to be added may be 1.0 µmol/L to 3.0 µmol/L, and within the above concentration range, the cell performance (the barrier function and the like) tends to be stable with good reproducibility.

In the proliferation step, the intestinal stem cells can be cultured preferably in the presence of a GSK-3β inhibitor, a histone deacetylation inhibitor, and a serum substitute, or in the presence of a GSK-3β inhibitor and a serum substitute.

Examples of the histone deacetylation inhibitor include valproic acid, vorinostat, trichostatin A, tubastatin A, givinostat, and pracinostat.

The serum substitute is a composition that is used as a substitute for serum containing a differentiation-inducing factor, in order to culture iPS cells, ES cells, or the like while maintaining the undifferentiated state thereof. Preferably, a Knockout serum replacement (KSR) is used.

An example of the concentration of the histone deacetylation inhibitor (in the case of valproic acid) to be added is 0.1 mmol/L to 10 mmol/L and preferably 0.5 mmol/L to 3 mmol/L, and the concentration of the serum substitute (in the case of KSR) to be added is 5% (v/v) to 20% (v/v) and preferably 5% (v/v) to 10% (v/v).

The proliferation step preferably can be carried out under the conditions in which one or more compounds selected from the group consisting of an epithelial cell growth factor (EGF), a TGFβ receptor inhibitor (such as A8301), a Rho-associated coiled-coil forming kinase (ROCK) inhibitor (such as Y-27632), and a fibroblast proliferation factor (such as FGF2) are further present. In the proliferation step, the concentration of EGF in the culture medium is, for example, 1.0 ng/mL to 1 mg/mL, and it is preferably 10 ng/mL to 300 ng/mL and more preferably 30 ng/mL to 300 ng/mL. The concentration of the TGFβ receptor inhibitor is, for example, 10 ng/mL to 10 µmol/L and preferably 0.1 µmol/L to 1.0 µmol/L. The concentration of the ROCK inhibitor is, for example, 0.1 µmol/L to 30 µmol/L and preferably 0.5 µmol/L to 10 µmol/L. For example, the concentration of FGF2 is 1.0 ng/mL to 100 ng/mL and preferably 10 ng/mL to 100 ng/mL.

The culture period of the proliferation step is not particularly limited; however, it is preferably 1 to 10 days, more preferably 1 to 5 days, and still more preferably 2 to 4 days, and an example thereof may be 3 days.

<Second differentiation step: differentiation into intestinal epithelial cell>

The second differentiation step is a step of differentiating the intestinal stem cells into intestinal epithelial cells. The second differentiation step preferably includes a step of differentiating the intestinal stem cells into the intestinal epithelial cell in the presence of a MEK1 inhibitor, a DNA methylation inhibitor, a TGFβ receptor inhibitor, EGF, and a cAMP activator.

In a case of using a cAMP activator, it is possible to positively increase the intracellular cAMP level. "In the presence of a MEK1 inhibitor, a DNA methylation inhibitor, a TGFβ receptor inhibitor, EGF, and a cAMP activator" refers to that all these compounds are required in order to carry out one or more cultures constituting the second differentiation step, and it is not required, as an essential condition, that all of these compounds are used at the same time, that is, that the culture using a culture medium to which all of these compounds are added is carried out.

Typically, the cell population obtained in the first differentiation step or a part thereof is subjected to the second differentiation step without sorting. On the other hand, intestinal stem cells may be sorted from the cell population obtained in the first differentiation step, and then the second differentiation step may be carried out. The sorting of intestinal stem cells may be carried out, for example, with a flow cytometer (a cell sorter) using a cell surface marker as an indicator.

The second differentiation step is constituted of one or two or more cultures (details will be described later). In each culture constituting the second differentiation step, culture media such as a culture medium to which EGF and a cAMP activator have been added as essential components, a culture medium to which a MEK1 inhibitor, a DNA methylation inhibitor, a TGFβ receptor inhibitor, and EGF have been added as essential components, a culture medium to which EGF has been added as an essential component, and a culture medium to which a MEK1 inhibitor, a DNA methylation inhibitor, a TGFβ receptor inhibitor, EGF, and a cAMP activator have been added as essential components are used.

Examples of the MEK1 inhibitor include PD98059, PD184352, PD184161, PD0325901, U0126, MEK inhibitor I, MEK inhibitor II, MEK1/2 inhibitor II, and SL327. Examples of the DNA methylation inhibitor include 5-aza-2'-deoxycytidine, 5-azacytidine, RG108, and zebularine. Regarding the TGFβ receptor inhibitor, considering that A8301 used in Examples described later exhibits an inhibitory activity on TGF-β receptors ALK4, ALK5, and ALK7, it is preferable to use an inhibitor that exhibits an inhibitory activity on one or more of TGF-β receptors, ALK4, ALK5, and ALK7. For example, A8301, SB431542, SB-505124, SB525334, D4476, ALK5 inhibitor, LY2157299, LY364947, GW788388, and RepSox satisfy the above conditions. As the cAMP activator, Forskolin, indomethacin, NKH477 (colforsin daropate), a cell-derived toxin protein (pertussis toxin, cholera toxin), PACAP-27, PACAP-38, SKF83822, or the like can be used. Forskolin exhibits an adenylate cyclase activating activity and promotes the intracellular cAMP synthesis.

An example of the concentration of the MEK1 inhibitor to be added (in the case of PD98059) is 4 µmol/L to 100 µmol/L and preferably 10 µmol/L to 40 µmol/L. An example of the concentration of the DNA methylation inhibitor (in the case of 5-aza-2'-deoxycytidine) to be added is 1 µmol/L to 25 µmol/L and preferably 2.5 µmol/L to 10 µmol/L, and an example of the concentration of the TGFβ receptor inhibitor (in the case of A8301) to be added is 0.1 μmol/L to 2.5 μmol/L and preferably 0.2 μmol/L to 1 μmol/L. An example of the concentration of EGF to be added is 5 ng/mL to 100 ng/mL and preferably 10 ng/mL to 50 ng/mL. An example of the concentration of the CAMP activator to be added (in the case of Forskolin) is 1 μmol/L to 200 μmol/L and preferably 5 μmol/L to 100 μmol/L. However, the concentration of compounds in a case of using compounds different from the exemplified compounds, that is, PD98059, 5-aza-2'-deoxycytidine, A8301, and Forskolin, can be set according to the above-described concentration range by those skilled in the art in consideration of the difference in the properties (especially the difference in activity) between the compound to be used and the exemplified compounds (PD98059, 5-aza-2'-deoxycytidine, A8301, and Forskolin). Whether the set concentration range is suitable or not can be confirmed by a preliminary experiment according to Examples described later.

In addition to the conditions described above, the step (2) may be carried out under the condition in which cAMP is supplied to cells (referred to as an "additional condition 1") and the condition in which a cAMP-degrading enzyme inhibitor is present (referred to as an "additional condition 2"), or under any of these conditions. The additional condition 1 (the condition in which cAMP is supplied to cells) is synonymous with the condition in which a compound capable of being incorporated into cells and acting as cAMP when taken up into cells is present. Accordingly, in order to satisfy the additional condition 1, for example, a culture medium to which a cAMP derivative that can be incorporated into cells is added may be used. In a case where the additional condition 1 is adopted, it can be expected that the decrease in the intracellular cAMP concentration is suppressed, and thus the induction of differentiation into intestinal epithelial cell, in particular, the acquisition of a function as intestinal epithelial cells is promoted. That is, the above condition may enable the preparation of more functional intestinal epithelial cells. As a cAMP derivative, it is possible to employ a PKA activator (for example, 8-Br-cAMP (a 8-bromoadenosine-3',5'-cyclic monophosphate sodium salt, CAS Number: 76939-46-3), 6-Bnz-cAMP (an N6-benzoyladenosine-3',5'-cyclic monophosphate soldium salt, CAS Number: 1135306-29-4), cAMPS-Rp (an (R)-adenosine cyclic 3',5'-(hydrogen phosphorothioate)triethlylamminium salt, CAS Numer: 151837-09-1), cAMPS-Sp (an (S)-adenosine cyclic 3',5'-(hydrogen phosphorothioate)triethylammanium salt, CAS Number: 93602-66-5), dibutyryl-cAMP (an N6,O2'-dibutyryladenosine 3',5'-cyclic monophosphate solidium salt, CAS Number: 16980-89-5), 8-Cl-cAMP (a 8-chloroadenosine-3',5'-cyclic monophosphate salt, CAS Number: 124705-03-9)), an Epac activator (Rp-8-Br-cAMPS (8-bromodenosine3',5',-cyclic monophosphoothioate, an Rp-isomer sodium salt, CAS Number: 129735-00-8), 8-CPT-cAMP (8-(4-chlorophenylthio)adenosine 3',5'-cyclic monophosphate, CAS Number: 93882-12-3), or 8-pCPT-2'-O-Me-cAMP (8-(4-chlorophenylthio)-2'-O-methyladenosine 3',5'-cyclic monophosphate monosodium, CAS Number: 634207-53-7), or the like). An example of the concentration of the cAMP derivative (in the case of 8-Br-cAMP) to be added is 0.1 mmol/L to 10 mmol/L, and it is preferably 0.2 mmol/L to 5 mmol/L and more preferably 0.5 mmol/L to 2 mmol/L. In addition, in a case of using a compound different from the exemplified compound, that is, 8-Br-cAMP, the addition concentration can be set according to the above-described concentration range by those skilled in the art in consideration of the difference in the properties (especially the difference in activity) between the compound used and the exemplified compound (8-Br-cAMP). Whether the set concentration range is suitable or not can be confirmed by a preliminary experiment according to Examples described later.

The additional condition 2 (the condition in which a cAMP-degrading enzyme inhibitor is present) is synonymous with the condition in which the cAMP-degrading enzyme inhibitor is added to the culture medium. In a case where the additional condition 2 is adopted, it can be expected that the decrease in the intracellular cAMP concentration is suppressed by the inhibition of cAMP degradation and thus the induction of differentiation into intestinal epithelial cell, in particular, the acquisition of a function as intestinal epithelial cells is promoted. That is, the above condition may enable the preparation of more functional intestinal epithelial cells. In addition, in a case where the additional conditions 1 and 2 are used in combination, it is possible to suppress the decrease in intracellular cAMP concentration while supplying cAMP to cells. As a result, the condition becomes effective for maintaining intracellular cAMP at a high level, and it can be expected that efficient induction of differentiation into intestinal epithelial cells is promoted.

Examples of the cAMP-degrading enzyme inhibitor include IBMX (3-isobutyl-1-methylxanthine) (MIX), Theophylline, Papaverine, Pentoxifilline (Trental), KS-505, 8-methoxymethyl-IBMX, Vinpocetine (TCV-3B), EHNA, Trequinsin (HL-725), Lixazinone (RS-82856), (LY-186126), Cilostamide (OPC3689), Bemoradan (RWJ-22867), Anergrelide (BL4162A), Indolidan (LY195115), Cilostazol (OPC-13013), Milrinone (WIN47203), Siguazodan (SKF-94836), 5-methyl-imazodan (CI 930), SKF-95654, Pirilobendan (UD-CG 115 BS), Enoximone (MDL 17043), Imazodan (CL 914), SKF-94120, Vesnarinone (OPC 8212), Rolipram (Ro-20-1724), (ZK-62711), Denbufyll'ine, Zaprinast (M&B-22,948), Dipyridamole, Zardaverine, and AH-21-132, Sulmazol (AR-L 115 BS). An example of the concentration of the cAMP-degrading enzyme inhibitor (in the case of IBMX) to be added is 0.05 mmol/L to 5 mmol/L, and it is preferably 0.1 mmol/L to 3 mmol/L and more preferably 0.2 mmol/L to 1 mmol/L. In addition, in a case of using a compound different from the exemplified compound, that is, IBMX, the addition concentration can be set according to the above-described concentration range by those skilled in the art in consideration of the difference in the properties (especially the difference in activity) between the compound used and the exemplified compound (IBMX). Whether the set concentration range is suitable or not can be confirmed by a preliminary experiment according to Examples described later.

**EP 4 174 170 A1**

The period (the culture period) of the second differentiation step is, for example, 7 days to 40 days and preferably 10 days to 30 days. In a case where the culture period is too short, an expected effect (increase in differentiation efficiency or promotion of acquisition of functions as intestinal epithelial cells) cannot be sufficiently obtained. On the other hand, in a case where the culture period is too long, the differentiation efficiency will be reduced.

The differentiation into intestinal epithelial cells can be determined or evaluated using, for example, the expression of an intestinal epithelial cell marker, the incorporation of a peptide, or the induction of the expression of a drug metabolizing enzyme via a vitamin D receptor as an indicator. Examples of the intestinal epithelial cell marker include ATP-binding cassette transporter B1/Multidrug resistance protein 1 (ABCB1/MDR1), ATP-binding cassette transporter G2/Breast cancer resistance protein (ABCG2/BCRP), cytochrome P4503A4 (CYP3A4), Fatty acid binding protein 2 (FABP2), Pregnane X receptor (PXR), Solute carrier (SLC) family member 5A1/Sodium-coupled glucose transporter 1 (SLC5A1/SGLT1), Solute carrier (SLC) family member 15A1/Peptide transporter 1 (SLC15A1/PEPT1), Solute carrier (SLC) organic anion transporter 2B1 (SLCO$_2$B1/OATP2B1), Sucrase-isomaltase, Uridine diphosphate glucuronosyltransferase 1A1 (UGT1A1), Villin 1, Uridine diphosphate glucuronosyltransferase 1A4 (UGT1A4), and Carboxylesterase 2A1 (CES2A1). Among these, Sucrase-isomaltase and Villin 1 which are highly specific to the intestinal epithelial cell, CYP3A4 which is a major drug metabolizing enzyme in the small intestine, SLC15A1/PEPT1 which is involved in peptide absorbance in the small intestine, SLC5A1/SGLT1, a glucose transporter, which is expressed at the apical membrane side of the small intestine, SLCO$_2$B1/OATP2B1 which is involved in the absorbance of organic anions in the small intestine, and CES2A1, a hydrolase, which is highly expressed in the small intestine, are particularly effective markers.

To obtain a cell population consisting only of target cells (intestinal epithelial cells) or a cell population including the target cells in a high proportion (high purity), a cell population after culture may be selected and sorted using a cell surface marker characteristic of the target cells as an indicator.

As the step (2), any one of the following culture steps A to D is preferably carried out.

<Culture step A>

In culture step A, a culturing (a-1) in the presence of EGF and an intracellular cAMP synthesis promoter and a culturing (a-2) in the presence of a MEK1 inhibitor, a DNA methylation inhibitor, a TGFβ receptor inhibitor, and EGF, which is carried out after the culturing (a-1). In a case of carrying out the two-stage culture in this manner, the effects of the promotion of differentiation into intestinal epithelial cells, maturation, and the acquisition of functions can be expected. The period of the culturing (a-1) is, for example, 2 days to 10 days and preferably 4 days to 8 days, and the period of the culturing (a-2) is, for example, 7 days to 29 days and preferably 9 days to 27 days. For items not particularly described (compounds usable for each culture, the concentration of each compound to be added, and the like), the corresponding description described above is cited.

The culturing (a-1) may be carried out under the condition in which cAMP is supplied to cells (referred to as "additional condition 1") and the condition in which a cAMP-degrading enzyme inhibitor is present (referred to as "additional condition 2"), or under any of these conditions. The same applies to the culturing (a-2). Details of the additional condition 1 and the additional condition 2 are as described above.

<Culture step B>

In culture step B, a culturing (b-1) in the presence of EGF and a culturing (b-2) in the presence of a MEK1 inhibitor, a DNA methylation inhibitor, a TGFβ receptor inhibitor, EGF, and an intracellular cAMP synthesis promoter, which is carried out after the above culture, are carried out. In a case of carrying out the two-stage culture in this manner, the effects of the promotion of differentiation into intestinal epithelial cells, maturation, and the acquisition of functions can be expected. The period of the culturing (b-1) is, for example, 2 days to 10 days and preferably 4 days to 8 days, and the period of the culturing (b-2) is, for example, 7 days to 19 days and preferably 9 days to 17 days. For items not particularly described (compounds usable for each culture, the concentration of each compound to be added, and the like), the corresponding description described above is cited.

The culturing (b-1) may be carried out under the condition in which cAMP is supplied to cells (an "additional condition 1") and the condition in which a cAMP-degrading enzyme inhibitor is present (an "additional condition 2"), or under any of these conditions. The same applies to the culturing (b-2). Details of the additional condition 1 and the additional condition 2 are as described above.

After the culturing (b-2), the culture (the culturing (b-3)) in the presence of a MEK1 inhibitor, a DNA methylation inhibitor, a TGFβ receptor inhibitor, and EGF may be carried out. The period of this culture is, for example, 1 day to 10 days. In a case of carrying out this culture, the effects of the promotion of differentiation into intestinal epithelial cells, maturation, and the acquisition of functions can be expected.

11

<Culture step C>

In culture step C, a culturing (c-1) in the presence of EGF and an intracellular cAMP synthesis promoter and a culturing (c-2) in the presence of a MEK1 inhibitor, a DNA methylation inhibitor, a TGFβ receptor inhibitor, EGF, and an intracellular cAMP synthesis promoter, which is carried out after the above culture, are carried out. In a case of carrying out the two-stage culture in this manner, the effects of the promotion of differentiation into intestinal epithelial cells, maturation, and the acquisition of functions can be expected. The period of the culturing (c-1) is, for example, 2 days to 10 days and preferably 4 days to 8 days, and the period of the culturing (c-2) is, for example, 7 days to 19 days and preferably 9 days to 17 days. For items not particularly described (compounds usable for each culture, the concentration of each compound to be added, and the like), the corresponding description described above is cited.

The culturing (c-1) may be carried out under the condition in which cAMP is supplied to cells (an "additional condition 1") and the condition in which a cAMP-degrading enzyme inhibitor is present (an "additional condition 2"), or under any of these conditions. The same applies to the culturing (c-2). Details of the additional condition 1 and the additional condition 2 are as described above.

After the culturing (c-2), the culture (the culturing (c-3)) in the presence of a MEK1 inhibitor, a DNA methylation inhibitor, a TGFβ receptor inhibitor, and EGF may be carried out. The period of this culture is, for example, 1 day to 10 days. In a case of carrying out this culture, the effects of the promotion of differentiation into intestinal epithelial cells, maturation, and the acquisition of functions can be expected.

<Culture step D>

In culture step D, a culturing (d-1) in the presence of a MEK1 inhibitor, a DNA methylation inhibitor, a TGFβ receptor inhibitor, EGF, and an intracellular cAMP synthesis promoter. This culture step is particularly advantageous in that the culture operation is simple, the differentiation into intestinal epithelial cells is more effective, and a stable effect can be expected since chemical compounds are used. The period of the culturing (d-1) is, for example, 15 days to 25 days and preferably 17 days to 23 days. For items not particularly described (compounds usable for each culture, the concentration of each compound to be added, and the like), the corresponding description described above is cited.

The culturing (d-1) may be carried out under the condition in which cAMP is supplied to cells (an "additional condition 1") and the condition in which a cAMP-degrading enzyme inhibitor is present (an "additional condition 2"), or under any of these conditions. Details of the additional condition 1 and the additional condition 2 are as described above.

After the culturing (d-1), the culture (the culturing (d-2)) in the presence of a MEK1 inhibitor, a DNA methylation inhibitor, a TGFβ receptor inhibitor, and EGF may be carried out. The period of this culture is, for example, 1 day to 10 days. In a case of carrying out this culture, the effects of the promotion of differentiation into intestinal epithelial cells, maturation, and the acquisition of functions can be expected.

In each step (the first differentiation step and a step constituting the first differentiation step, the proliferation step, and the second differentiation step and a step constituting the second differentiation step) in the present invention, subculture may be carried out in the middle of each step. For example, in a case where cells become confluent or subconfluent, a part of the cells are collected and transferred to another culture container, and the culture is continued. It is preferable to set a cell density low in order to promote differentiation. For example, cells may be seeded at a cell density of about $1 \times 10^4$ cells/cm$^2$ to $1 \times 10^6$ cells/cm$^2$.

In a case of recovering cells in association with culture medium exchange or subculture, cells may be treated in advance with a ROCK (Rho-associated coiled-coil forming kinase) inhibitor such as Y-27632 to suppress cell death.

Other culture conditions (such as the culture temperature) in the individual steps constituting the present invention may be conditions generally employed in culturing animal cells. That is, culture may be carried out, for example, at 37°C in an environment of 5% CO$_2$. In addition, as a basal medium, Iskov modified Dulbecco's culture medium (IMDM) (GIBCO-BRL or the like), Ham F12 culture medium (Ham F12) (SIGMA, Gibco-BRL, or the like), Dulbecco's modified Eagle's culture medium (D-MEM) (Nacalai Tesque Inc., Sigma-Aldrich Co. LLC, Gibco-BRL or the like), Glasgow basal medium (Gibco-BRL or the like), RPMI1640 culture medium, or the like can be used. Two or more basal media may be used in combination. In the step (1-2) and the second differentiation step (as well as the culture step A, the culture step B, the culture step C, and the culture step D, which constitute the second differentiation step), a basal medium suitable for culturing epithelial cells (for example, a mixed culture medium of D-MEM and Ham F12 medium, and D-MEM) can be preferably used. Examples of components that can be added to the culture medium include bovine serum albumin (BSA), an antibiotic, 2-mercaptoethanol, PVA, non-essential amino acids (NEAA), insulin, transferrin, and selenium. Typically, cells are two-dimensionally cultured using a culture dish or the like. According to the method of the embodiment of the present invention, an intestinal epithelial cell-like cell can be obtained from pluripotent stem cells by two-dimensional culture. In addition, three-dimensional culture may be carried out using a gel-like culture base material or a three-dimensional culture plate.

In an example, the proliferation step and the second differentiation step can be carried out in a culture container coated with an extracellular matrix of 3% by volume to 100% by volume.

In another example, the proliferation step and the second differentiation step can be carried out in a culture medium to which an extracellular matrix of 1% by 10% by volume is added. In this case, the proliferation step and the second differentiation step may be carried out in a culture medium in which the extracellular matrix of 1% by volume to 10% by volume is added to only a basolateral side of the cell. As the example of the extracellular matrix, it is possible to use Matrigel (registered trade name), fibronectin, laminin, vitronectin, tenascin, entactin, thrombospondin, elastin, gelatin, collagen, fibrin, merosin, ankyrin, chondronectin, link protein, bone sialoprotein, osteocalcin, osteopontin, epinectin, hyaluronectin, undulin, epiregulin, or kalinin can be used, where Matrigel (registered trade name) is preferable used. Matrigel (registered trade name) is a product name of a gelatinous protein mixture secreted by Engelbreth-Holm-Swarm mouse sarcoma cells produced by Corning Life Sciences.

<Intestinal epithelial cell and cell sheet>

According to the present invention, there are provided An intestinal epithelial cell that is produced by the method according to the embodiment of the present invention, and a cell sheet containing the intestinal epithelial cells.

In the cell sheet containing the intestinal epithelial cells derived from a pluripotent stem cell according to the present invention, the proportion of columnar intestinal epithelial cells in the intestinal epithelial cells is 10% or more, preferably 15% or more, and more preferably 20% or more. The proportion of columnar intestinal epithelial cells in the intestinal epithelial cells may be 50% or more, 60% or more, 70% or more, 80% or more, 90% or more, or and it may be 100%. The columnar intestinal epithelial cell means an intestinal epithelial cell in which the longest axis of the cell is 3 times or more with respect to the longest axis of the nucleus. In addition, the columnar intestinal epithelial cell preferably satisfies $v/h \geq 1$ in a case where the length of the cell in the direction horizontal to the adhesion surface of the basolateral part of the cell is denoted by $h$ and the length of the cell in the direction perpendicular to the adhesion surface is denoted by $v$.

In the cell sheet according to the embodiment of the present invention, the cell density is preferably $50 \times 10^4$ cells/cm$^2$ or more, more preferably $60 \times 10^4$ cells/cm$^2$ or more, still more preferably $65 \times 10^4$ cells/cm$^2$ or more, and particularly preferably $70 \times 10^4$ cells/cm$^2$ or more. The cell density may be $75 \times 10^4$ cells/cm$^2$ or more or $80 \times 10^4$ cells/cm$^2$ or more.

The amount of the midazolam metabolite per area of the cell sheet is preferably 300 pmol/cm$^2$ or more, more preferably 360 pmol/cm$^2$ or more, and still more preferably 420 pmol/cm$^2$ or more. The amount of midazolam metabolite per area of the cell sheet may be 450 pmol or more, 540 pmol or more, 600 pmol or more, or 630 pmol or more.

The amount of the midazolam metabolite per area of the cell sheet is an amount of the midazolam metabolite after incubation in a culture medium containing 5 $\mu$mol/L midazolam at 37°C for 2 hours.

In the cell sheet, the CYP3A4 activity per protein mass is preferably 400 to 2,500 pmol/2 hr/mg·protein, and it is more preferably 500 to 2,000 pmol/2 hr/mg·protein. The CYP3A4 activity per protein mass may be 700 to 2,000 pmol/2 hr/mg·protein, 1,000 to 2,000 pmol/2 hr/mg·protein, or 1,000 to 1,500 pmol/2 hr/mg·protein.

The CYP3A4 activity per protein mass can be determined by adding a culture medium containing a substrate (midazolam), quantifying an amount of a metabolite (1'-hydroxymidazolam) thereof in a supernatant recovered after a certain period of time, and dividing the amount by the protein mass. The amount of the metabolite in the supernatant can be measured using a liquid chromatography-mass spectrometer (LC-MS/MS) or the like.

In general, the apparent permeability coefficient can be used as an indicator for predicting pharmacokinetics. The apparent permeability coefficient of midazolam in the cell sheet is preferably $40 \times 10^{-6}$ cm/s or less, more preferably $38 \times 10^{-6}$ cm/s or less, still more preferably $35 \times 10^{-6}$ cm/s or less, and particularly preferably $33 \times 10^{-6}$ cm/s or less. The apparent permeability coefficient of midazolam can be determined by adding midazolam to the apical side of the cell and quantifying the amount of midazolam that has permeated to the basolateral side after a certain period of time. The amount of midazolam that has permeated can be measured using a liquid chromatography-mass spectrometer (LC-MS/MS) or the like. The apparent permeability coefficient of midazolam determined in vitro using a small intestinal tissue of a living body is $5 \times 10^{-6}$ cm/s (A-L. Ungel et al., Eur. J. Phrm. Sci. 48, 166 (2013)).

<Use application of intestinal epithelial cell and cell sheet>

The present invention relates to the use application of the intestinal epithelial cell or cell sheet produced by the method according to the embodiment of the present invention.

As a first use application, there are provided an evaluation method (a screening method) for a test substance, including bringing a test substance into contact with the intestinal epithelial cell or the cell sheet according to the embodiment of the present invention, and a screening kit for a test substance, including the intestinal epithelial cell or the cell sheet according to the embodiment of the present invention. The intestinal epithelial cell or the cell sheet of the embodiment of the present invention can be used for a model system of the intestinal tract, particularly the small intestine, and it is useful for the evaluation of pharmacokinetics (absorbance, metabolism, and the like) and the evaluation of toxicity in the intestinal tract, particularly the small intestine, and the response evaluation for the small intestine. In other words, the intestinal epithelial cell or the cell sheet according to the embodiment of the present invention can be used for the evaluation of in vivo pharmacokinetics of a test substance, the evaluation of toxicity, and the evaluation of response to a test substance.

Specifically, the intestinal epithelial cell or the cell sheet of the embodiment of the present invention can be used to test the absorbability or membrane permeability, drug interaction, induction of a drug metabolizing enzyme, induction of a drug transporter, toxicity, immune response, or the like with respect to the test substance. That is, the present invention provides a method (a first aspect) for evaluating absorbability or membrane permeability, drug interaction, induction of a drug metabolizing enzyme, induction of a drug transporter, toxicity, immune response, or the like with respect to the test substance as one of the use applications of the intestinal epithelial cell and the cell sheet. The above-described method carries out a step (i) of preparing a cell layer formed of the intestinal epithelial cell-like cells obtained by the differentiation induction method of the embodiment of the present invention, a step (ii) of bringing a test substance into contact with the cell layer; and a step (iii) of quantifying the test substance that has permeated through the cell layer and evaluating absorbability or membrane permeability, drug interaction, induction of a drug metabolizing enzyme, induction of a drug transporter, immune response, or toxicity of the test substance. In addition, the absorbability of the test substance can also be evaluated by the method described below (second aspect).

In the step (i), intestinal epithelial cell-like cells are typically cultured on a semipermeable membrane (porous membrane) to form a cell layer. Specifically, for example, by using a culture container equipped with a cell culture insert (for example, Transwell (registered trade name) provided by Corning Incorporated), cells are seeded and cultured in the cell culture insert, and then a cell layer constituted of the intestinal epithelial cell-like cells are obtained.

The "contact" in the step (ii) is typically carried out by adding a test substance to a culture medium. The timing for adding the test substance is not particularly limited. Accordingly, the test substance may be added at a certain timing after starting culture in a culture medium containing no test substance, or the culture may be started in a culture medium containing the test substance in advance.

As the test substance, organic compounds or inorganic compounds having various molecular sizes can be used, and bacteria (enteric bacteria, lactic acid bacteria, and the like), viruses, and cells (immune cells, fibroblasts) can also be used. Examples of the organic compound include a nucleic acid, a peptide, a protein, a lipid (a simple lipid, a complex lipid (a phosphoglyceride, a sphingolipid, a glycosylglyceride, a cerebroside, or the like), a prostaglandin, an isoprenoid, a terpene, a steroid, a polyphenol, catechin, and a vitamin (B 1, B2, B3, B5, B6, B7, B9, B12, C, A, D, E, or the like). Existing components or candidate components such as a pharmaceutical drug, a nutritional food product, a supplement, a food additive, a pesticide, and perfumery (a cosmetic) are also one of the preferable test substances. A plant extract, a cell extract, a culture supernatant or the like may be used as the test substance. By adding two or more test substances at the same time, the interaction, the synergism, or the like between the test substances may be examined. The test substance may be of natural origin or synthetic. In the latter case, an efficient assay system can be constructed using, for example, a combinatorial synthesis technique.

The period for bringing the test substance into contact can be appropriately set. The contact period is, for example, 10 minutes to 3 days and preferably 1 hour to 1 day. The contact may be carried out a plurality of times.

In the step (iii), the test substance that has permeated through the cell layer is quantified. For example, in a case where a culture container equipped with a cell culture insert such as Transwell (registered trade name) is used, a test substance that has permeated through the cell culture insert, that is, the test substance that has moved into the upper or lower vessel via the cell layer is quantified depending on the test substance by a measuring method such as mass spectrometry, liquid chromatography, and immunological techniques (for example, a fluorescent immunoassay method (an FIA method) and an enzymatic immunoassay method (an EIA method)). Based on the quantification results (the amount of the test substance that has permeated through the cell layer) and the amount of the test substance used (typically, the amount added to the culture medium), the absorbability or membrane permeability, drug interaction, induction of a drug metabolizing enzyme, induction of a drug transporter, immune response, or toxicity are determined and evaluated with respect to the test substance.

The present invention also provides, as another aspect (second aspect), a method for evaluating the metabolism or absorbance of a test substance. In the above-described method, a step (I) of bringing a test substance into contact with the intestinal epithelial cell-like cells obtained by the differentiation induction method of the embodiment of the present invention and a step (II) of measuring and evaluating metabolism or absorbance, drug interaction, induction of a drug metabolizing enzyme, induction of a drug transporter, immune response, or toxicity of the test substance are carried out.

The step (I), that is, the bringing of the test substance into contact with intestinal epithelial cell-like cells can be carried out in the same manner as in the step (ii). However, it is not essential to form a cell layer in advance.

After the step (I), the metabolism or absorbance, drug interaction, induction of a drug metabolizing enzyme, induction of a drug transporter, toxicity, or immune response is measured and evaluated with respect to the test substance (a step (II)). Metabolism or the like may be measured and evaluated, substantially without time interval, immediately after the step (I), that is, after bringing the test substance into contact with cells, or the metabolism or the like may be measured and evaluated after a certain time (for example, 10 minutes to 5 hours) has passed. The measurement of metabolism can be carried out, for example, by detecting a metabolite. In this case, the expected metabolite is usually qualitatively or quantitatively measured using the culture solution after the step (I) as a sample. A suitable measuring method may be selected depending on the metabolite, and for example, mass spectrometry, liquid chromatography, and an immunological method (for example, a fluorescent immunoassay method (FIA method) and an enzymatic immunoassay method (EIA method)), or the like can be employed.

Typically, in a case where a metabolite of a test substance is detected, it is determined or evaluated that "the test substance has been metabolized". In addition, the metabolism quantity of the test substance can be evaluated depending on the amount of the metabolite. The metabolism efficiency of the test substance may be calculated based on the detection result of the metabolite and the used amount of the test substance (typically, the amount added to the culture medium).

The metabolism of a test substance can be measured using, as an indicator, the expression of drug metabolizing enzymes (cytochrome P450 (particularly CYP3A4), uridine diphosphate glucuronosyltransferase (particularly UGT1A8 or UGT1A10), and sulfotransferase (particularly SULT1A3 or the like)) in intestinal epithelial cell-like cells. The expression of drug metabolizing enzymes can be evaluated at the mRNA level or the protein level. For example, in a case where an increase in the mRNA level of a drug metabolizing enzyme is recognized, it can be determined that "the test substance has been metabolized". Similarly, in a case where an increase in the activity of a drug metabolizing enzyme is recognized, it can be determined that "the test substance has been metabolized". Similarly to the case where the metabolite is used as an indicator for determination, quantitative determination or evaluation may be carried out based on the expression level of the drug metabolizing enzyme.

In order to evaluate the absorption of a test substance, for example, the remaining amount of the test substance in the culture solution is measured. Usually, the test substance is quantified using the culture solution after the step (I) as a sample. A suitable measuring method may be selected depending on the test substance. For example, mass spectrometry, liquid chromatography, and an immunological method (for example, a fluorescent immunoassay method (FIA method) and an enzymatic immunoassay method (EIA method)), or the like can be employed. Typically, in a case where a decrease in the content of the test substance in the culture solution is recognized, it is determined or evaluated that "the test substance has been absorbed". In addition, the absorption amount or the absorption efficiency of the test substance can be determined or evaluated depending on the degree of the decrease. The absorption can also be evaluated by measuring the amount of the test substance incorporated into the cells.

The measurement or evaluation of the metabolism and the measurement or evaluation of the absorbance may be carried out simultaneously or in parallel.

In a case of using a scaling factor, it is possible to correct the evaluation result of the metabolism or absorption of the test substance serving as a substrate of a metabolic enzyme and improve the prediction accuracy.

Examples of the metabolic enzyme include cytochrome P450 (CYP). Examples of the cytochrome P450 (CYP) include CYP1A2, CYP2C9, CYP2C19, CYP2D6, and CYP3A4. In addition, examples of the metabolic enzyme include carboxyl esterase (CES), UDP-glucuronic acid transferase (UGT), sulfate transferase (SULT), and Sucrase-Isomaltase. Examples of the carboxyl esterase (CES) include CES1 and CES2. Examples of the UDP-glucuronic acid transferase (UGT) include UGT1A1, UGT1A6, UGT1A7, UGT2B7, and UGT2B11. Examples of the sulfate transfer enzyme (SULT) include SULT1A1, SULT1A3, SULT1B1, SULT1E1, and SULT2A1.

Examples of the test substance serving as a substrate of CYP3A4 include midazolam, verapamil, and tacrolimus.

The scaling factor is a constant obtained by focusing on the morphology (the cell morphology/tissue structure) and function (metabolic enzyme activity/transporter activity) of the small intestine of a living body. Various parameters obtained by allowing a test substance to pass through the cell or cell sheet according to the embodiment of the present invention and to be metabolized can be multiplied by the scaling factor.

The various parameters include the following five kinds.

1) Amounts of the test substance and the metabolite thereof in the test solution (on the apical side and the basolateral side), in the cells, and on the insert membrane.

2) A temporal change in 1).

3) All parameters obtained by calculation from 1) and 2).

For example, the apparent permeability coefficient, the permeation clearance, the metabolic clearance, or the efflux ratio.

4) Amounts of change in 1), 2), and 3) in a case where a drug that inhibits or induces metabolism or absorption is added.

5) Parameters which are obtained by calculation from 1), 2), 3), and 4), obtained by carrying out independent tests on two or more kinds of drugs.

In an example of the present invention, an apparent permeability coefficient can be corrected by using a scaling factor.

The apparent permeability coefficient Papp is obtained according to the following expression.

$$\text{Papp} = \text{the permeation amount per unit time/(the A0 concentration} \times \text{the permeation area)}$$

$$\text{The permeation amount per unit time} = \text{the B60 substrate amount/the permeation time}$$

The A0 concentration: The concentration in a case where the test substance serving as a substrate is added to the A side

The permeation area: The area through which the test substance permeates

The B60 substrate amount: The mass of the substrate permeated to the B side 60 minutes after the test substance serving as a substrate was added to the A side.

The permeation time: the time taken from the addition of the test substance serving as a substrate to the A side until the completion of sampling

In a case of using the scaling factor x, it is possible to correct, for example, the apparent permeability coefficient according to the following expression.

As a certain parameter in the permeation amount per unit time = (the B60 substrate amount - x × the certain parameter)/the permeation time, it is possible to use parameters described in 1), 2), 3), 4), and 5), and examples thereof include the parameters such as the amount of the metabolite and the difference in "the B60 substrate amount" due to the presence or absence of the addition of the metabolism inhibitor. In a case of using the parameter described in 2) above described, it is possible to use, as a parameter, a value obtained by multiplying time by the temporal change in 1) described above.

From the above, by correcting the apparent permeability coefficient, it is possible to improve the prediction accuracy of Fa × Fg or Fg in the small intestine of a living body. It is noted that Fa is an absorption rate in the intestinal tract, and for example, Fa of midazolam in the small intestine of a living body is considered to be 1.0 (document information: Takenaka T. et al. Drug Metab Dispos. 42, 1947, 54 (2014)), and Fg is the rate of avoidance of metabolism in the intestinal tract, and it is considered to be 0.369 to 0.5 (Pharm Res. 2012 Mar; 29 (3): 651).

As another expression, it is possible to establish an expression that makes it possible to simply determine Fg (Takayama K. et al. Cell. Mol. Gastroenterol. Hepatol., 8, 513-526 (2019)).

$$\text{Fg} = \text{(the B60 substrate amount} + \text{the cell 60 substrate amount)/(the B60 substrate amount} + \text{cell 60 substrate amount} + \text{the total 60 metabolite amount)} \cdots \text{(Expression 1)}$$

The B60 substrate amount: The mass of the substrate permeated to the B side 60 minutes after the test substance serving as a substrate was added to the A side.

The cell 60 substrate amount: The mass of the substrate accumulated in the cells 60 minutes after the test substance serving as a substrate was added to the A side.

The total 60 metabolite amount: Total amount of the metabolite accumulated on the A, on the B side, and in the cells 60 minutes after the test substance serving as a substrate was added to the A side.

An example of the parameter is "the total 60 metabolite amount" in the expression formula of Fg.

For example, the following is possible, Fg = 431/(431+91 × x), where x is a scaling factor. x can be set to 0.1 to 500, and it is preferably 1.2 to 30.

In an example of the present invention, Fg can be corrected by using a scaling factor.

As a second use application of the intestinal epithelial cell or the cell sheet according to the embodiment of the present invention, there is provided a cell preparation containing the intestinal epithelial cell or the cell sheet. The cell preparation according to the embodiment of the present invention can be applied to the treatment of various intestinal diseases. In particular, use as a material for regeneration/reconstruction of a damaged intestinal epithelial tissue (including dysfunction) is considered. That is, the contribution to regenerative medicine can be expected. The cell preparation of the embodiment of the present invention can be prepared by, for example, suspending the intestinal epithelial cell or the cell sheet of the embodiment of the present invention in a physiological saline solution or a buffer solution (for example, a phosphate buffer solution) or producing a three-dimensional tissue (organoid or spheroid) using the intestinal epithelial cell-like cells. In order to be able to administer a therapeutically effective amount of cells, a single dose may contain, for example, $1 \times 10^5$ to $1 \times 10^{10}$ cells. The cell content can be suitably adjusted in consideration of the purpose of use, the target disease, the gender, age, weight, and state of the affected part of the target (recipient) to be administered, cell state.

Dimethyl sulfoxide (DMSO) and serum albumin for the protection of cells, an antibiotic for the prevention of bacterial contamination, and various components (a vitamin, a cytokine, a growth factor, a steroid, and the like) for the activation, proliferation, induction of differentiation, or the like of cells may be contained in the cell preparation according to the embodiment of the present invention. In addition, other pharmaceutically acceptable components (for example, a carrier, an excipient, a disintegrant, a buffering agent, an emulsifying agent, a suspending agent, a soothing agent, a stabilizer, a preservative, an antiseptic agent, a physiological saline solution, and the like) may be contained in the cell preparation according to the embodiment of the present invention.

As a third use application of the intestinal epithelial cell or the cell sheet according to the embodiment of the present invention, culturing a microorganism (a bacterium, a fungus, a virus, or the like) or a parasite using the intestinal epithelial cell or the cell sheet according to the embodiment of the present invention can be mentioned. In a case of culturing a microorganism or a parasite using the intestinal epithelial cell or the cell sheet according to the embodiment of the present invention, it is possible to analyze the infectivity of the microorganism or the parasite to the intestinal epithelial cell. In addition, in a case where the infectivity is present, it is possible to check the maintenance or proliferation ability of the microorganism or the parasite. In addition, in a case of culturing a microorganism or a parasite using the intestinal epithelial cell or the cell sheet according to the embodiment of the present invention, it is possible to obtain a microorganism or a parasite, which proliferates repeatedly. Furthermore, it is possible to reproduce and utilize the infectious state of a pathogenic microorganism for the screening of a therapeutic drug or reproduce the effect of a substance produced by a microorganism or a parasite on the intestinal cells to screen for substances useful or harmful to the small intestine of a living body.

Specific examples of the microorganism or parasite include Escherichia coli, a lactic acid bacterium, a bifidus bacterium, an enteritis vibrio bacterium, a salmonella bacterium, various intestinal bacteria, an influenza virus, a rotavirus, an adenovirus, an astrovirus, a Sapporo virus, norovirus, an HIV, and a coronavirus, which are not limited thereto.

As a fourth use application of the intestinal epithelial cell or the cell sheet according to the embodiment of the present invention includes a co-culture model using the intestinal epithelial cell or the cell sheet according to the embodiment of the present invention can be mentioned. According to the co-culture model described above, it is possible reproduce the structure of the biological tissue and analyze the interaction with other cells. In addition, according to the co-culture model described above, it is possible to reproduce the pathological condition of a disease and carry out screening for a therapeutic drug.

The cell to be co-cultured is a cell species that is present in a living body of a primate or a rodent, and it is particularly preferably a cell that is present in the vicinity of the small intestine. Examples thereof include, but are not limited to, a hepatocyte, pancreas, a vascular endothelial cell, an immune cell, nerve, a fibroblast, a muscle cell, and a mesenchymal cell. The cell to be co-cultured may be a primate or rodent-derived established cell line. Examples thereof include, but are not limited to, an immortalized cell line such as a Hela cell and a THP-1 cell, a pluripotent stem cell such as an ES cell or an iPS cell, and a cell differentiated from the pluripotent stem cells.

As the test substance that can be tested in the co-culture model, organic compounds or inorganic compounds having various molecular sizes can be used. Existing components or candidate components such as medicines and nutritional food products are also preferred test substances. A plant extract, a cell extract, a culture supernatant or the like may be used as the test substance. In addition, two or more kinds of these may be used in combination.

Examples

<Culture of human iPS cell>

An iPS cell (an FF-1 strain provided from FUJIFILM Cellular Dynamics, Inc.) prepared from a human fibroblast according to the method described in Yu J, et al: Science 318 (5858), 1917-1920, 2007) was seeded on a 6-well plate or a 100 mm dish coated with Matrigel (registered trade name) and cultured at 37°C in a $CO_2$ incubator under the condition of 5% $O_2$. The subculture of the human iPS cell (the FF-1 strain) was carried out at a split ratio of 1:3 to 1:10 after culturing for 3 to 5 days. The culture medium was not changed for 48 hours after the subculture but was changed daily thereafter.

<Induction of human iPS cell differentiation into intestinal epithelial cell>

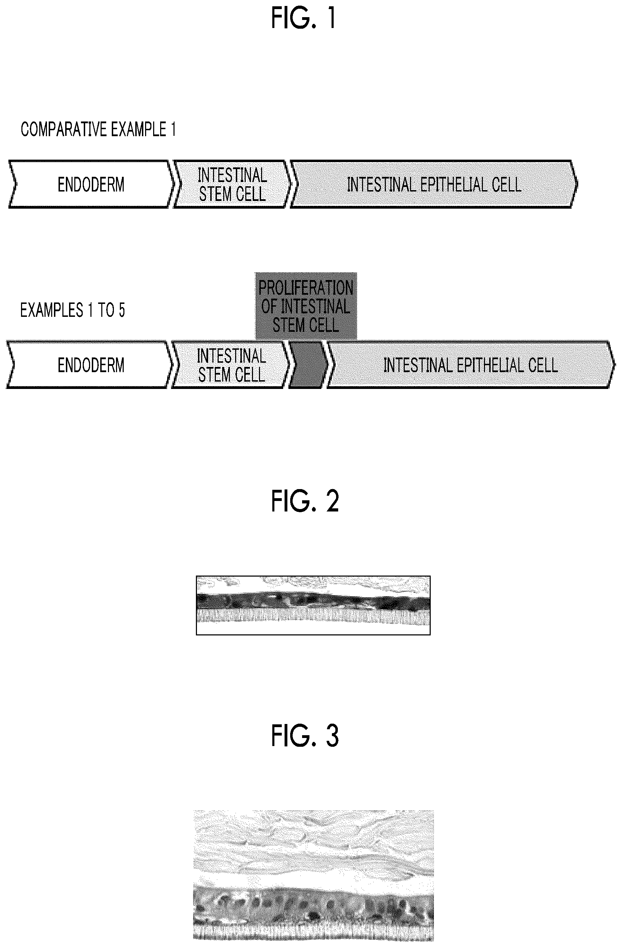

Fig. 1 illustrates an outline of the differentiation culture methods of Comparative Example 1 and Examples 1 to 5.

Comparative Example 1

The human iPS cell (the FF-1 strain) were cultured according to the method disclosed in WO2014/165663A by the culture in the presence of Activin A and the culture in the presence of BMP4, VEGF, FGF2, and EGF for a total of 7 days (from the 0th day to the 7th day), and further cultured in DMEM/F12 containing 2% FBS, 1% Glutamax, and 250 ng/mL FGF2 for 4 days (from the 7th day to the 11th day) and induced to be differentiated into intestinal stem cells.

Thereafter, Y-27632 (a Rho-binding kinase inhibitor) was added so that the concentration thereof was 10 $\mu$mol/L, and the cells were treated at 37°C for 60 minutes in a $CO_2$ incubator. The treated cells were detached by treatment with accutase and plated at a cell density of $2.0 \times 10^5$ cells/cm$^2$ on a translucent membrane on a cell culture insert (Falcon, # 353495) coated in advance with Matrigel (registered trade name). Further, the cells were cultured in DMEM/F12 containing 2% FBS, 1% Glutamax, 1% NEAA, 2% B27 supplement, 1% N2 supplement, 100 units/mL penicillin G, 100 $\mu$g/mL streptomycin, 20 ng/mL epithelial cell growth factor (EGF), and 10 $\mu$mol/L Y-27632 for one day (from the 11th to the 12th day). Further, the cells were cultured in a culture medium containing 2% FBS, 1% Glutamax, 1% NEAA, 2% B27 supplement, 1% N2 supplement, 100 units/mL penicillin G, 100 $\mu$g/mL streptomycin, 20 ng/mL epithelial cell growth factor (EGF), and 30 $\mu$mol/L Forskolin for 18 days, thereby inducing differentiation into intestinal epithelial cells. Then, 5 $\mu$mol/L 5-aza-2'dc, 20 $\mu$mol/L PD98059, and 0.5 $\mu$mol/L A8301 were further added to the above-described culture medium, and the culture was carried out for 13 days (from the 18th to the 31st days) to obtain intestinal epithelial cells.

Example 1

The human iPS cell (the FF-1 strain) were cultured for 11 days under the same conditions as in Comparative Example 1 and induced to be differentiated into intestinal stem cells.

Then, the cells were detached under the same conditions as in Comparative Example 1 and plated at a cell density of $2.0 \times 10^5$ cells/cm$^2$ on a translucent membrane on a cell culture insert (Falcon, # 353495) coated in advance with Matrigel (registered trade name). Further, the cells were cultured in DMEM/F12 containing 10% KSR, 1% Glutamax, 100 units/mL penicillin G, 100 $\mu$g/mL streptomycin, 100 ng/mL epithelial cell growth factor (EGF), 30 ng/mL FGF2, 2 $\mu$mol/L Y-27632, 0.5 $\mu$mol/L A8301, and 3 $\mu$mol/L CHIR99021 for 3 days (from the 11th to the 14th day).

Then, the culture was carried out for 6 days (from the 14th to the 20th days) under the same conditions as in Comparative Example 1 and then further carried out for 17 days (from the 20th to the 37th day) by adding 5-aza-2'dc, PD98059, and A8301 as in Comparative Example 1, thereby obtaining intestinal epithelial cells.

Example 2

The human iPS cell (the FF-1 strain) were cultured for 11 days under the same conditions as in Comparative Example 1 and induced to be differentiated into intestinal stem cells.

Then, the cells were detached under the same conditions as in Comparative Example 1 and plated at a cell density of $2.0 \times 10^5$ cells/cm$^2$ on a translucent membrane on a cell culture insert (Falcon, # 353495) coated in advance with Matrigel (registered trade name). Further, the cells were cultured in DMEM/F12 containing 10% KSR, 1% Glutamax, 100 units/mL penicillin G, 100 $\mu$g/mL streptomycin, 100 ng/mL epithelial cell growth factor (EGF), 30 ng/mL FGF2, 2 $\mu$mol/L Y-27632, 0.5 $\mu$mol/L A8301, and 500 ng/mL Wnt3a for 3 days (from the 11th to the 14th day).

Then, the culture was carried out for 6 days (from the 14th to the 20th days) under the same conditions as in Comparative Example 1 and then further carried out for 17 days (from the 20th to the 37th day) by adding 5-aza-2'dc, PD98059, and A8301 as in Comparative Example 1, thereby obtaining intestinal epithelial cells.

Example 3

The human iPS cell (the FF-1 strain) were cultured for 11 days under the same conditions as in Comparative Example 1 and induced to be differentiated into intestinal stem cells.

Then, the cells were detached under the same conditions as in Comparative Example 1 and plated at a cell density of $2.0 \times 10^5$ cells/cm$^2$ on a translucent membrane on a cell culture insert (Falcon, # 353495) coated in advance with Matrigel (registered trade name). Further, the cells were cultured in DMEM/F12 containing 10% KSR, 1% Glutamax, 100 units/mL penicillin G, 100 $\mu$g/mL streptomycin, 100 ng/mL epithelial cell growth factor (EGF), 30 ng/mL FGF2, 2 $\mu$mol/L Y-27632, 0.5 $\mu$mol/L A8301, and 5 $\mu$mol/L TWS119 for 3 days (from the 11th to the 14th day).

Then, the culture was carried out for 6 days (from the 14th to the 20th days) under the same conditions as in Comparative Example 1 and then further carried out for 17 days (from the 20th to the 37th day) by adding 5-aza-2'dc, PD98059, and A8301 as in Comparative Example 1, thereby obtaining intestinal epithelial cells.

Example 4

The human iPS cell (the FF-1 strain) were cultured for 11 days under the same conditions as in Comparative Example 1 and induced to be differentiated into intestinal stem cells.

Then, the cells were detached under the same conditions as in Comparative Example 1 and plated at a cell density of $2.0 \times 10^5$ cells/cm$^2$ on a translucent membrane on a cell culture insert (Falcon, # 353495) coated in advance with Matrigel (registered trade name). Further, the cells were cultured in DMEM/F12 containing 10% KSR, 1% Glutamax, 100 units/mL penicillin G, 100 $\mu$g/mL streptomycin, 100 ng/mL epithelial cell growth factor (EGF), 30 ng/mL FGF2, 2 $\mu$mol/L Y-27632, 0.5 $\mu$mol/L A8301, and 5 $\mu$mol/L BIO for 3 days (from the 11th to the 14th day).

Then, the culture was carried out for 6 days (from the 14th to the 20th days) under the same conditions as in Comparative Example 1 and then further carried out for 17 days (from the 20th to the 37th day) by adding 5-aza-2'dc, PD98059, and A8301 as in Comparative Example 1, thereby obtaining intestinal epithelial cells.

Example 5

The human iPS cell (the FF-1 strain) were cultured for 11 days under the same conditions as in Comparative Example 1 and induced to be differentiated into intestinal stem cells.

Then, the cells were detached under the same conditions as in Comparative Example 1 and plated at a cell density of $2.0 \times 10^5$ cells/cm$^2$ on a translucent membrane on a cell culture insert (Falcon, # 353495) coated in advance with Matrigel (registered trade name). Further, the cells were cultured in DMEM/F12 containing 10% KSR, 1% Glutamax, 100 units/mL penicillin G, 100 $\mu$g/mL streptomycin, 100 ng/mL epithelial cell growth factor (EGF), 30 ng/mL FGF2, 2 $\mu$mol/L Y-27632, 0.5 $\mu$mol/L A8301, and 2.5 $\mu$mol/L BIO for 3 days (from the 11th to the 14th day).

Then, the culture was carried out for 6 days (from the 14th to the 20th days) under the same conditions as in Comparative Example 1 and then further carried out for 17 days (from the 20th to the 37th day) by adding 5-aza-2'dc, PD98059, and A8301 as in Comparative Example 1, thereby obtaining intestinal epithelial cells.

In Comparative Example 1 and Examples 1 to 5, the cell density was $1.2 \times 10^5$ cells/cm$^2$ at the time when the human iPS cell (the FF-1 strain) was cultured for 11 days.

<Test Example 1>

The cell density of the intestinal stem cells obtained in the middle of Comparative Examples 1 and Examples 1 to 3 was evaluated. The intestinal stem cells obtained in Comparative Example 1 (on the 12th day) and Examples 1 to 3 (on the 14th day) were washed with D-PBS, and immersed in ice-cold methanol to be subjected to fixation and membrane permeation treatment. Then, DAPI was added thereto, followed by staining at room temperature for 5 minutes and replacement with D-PBS, and then observation was carried out with a fluorescence microscope. The cell density was calculated by counting the number of nuclei stained with DAPI. The results are shown in Table 1. As compared with Comparative Example 1, Examples 1 and 3 showed a high density of intestinal stem cells.

[Table 1]

|  | Cell density of intestinal stem cells [$\times 10^4$ cells/cm$^2$] |
|---|---|
| Comparative Example 1 | 13.6 |
| Example 1 | 45.3 |
| Example 2 | 32.2 |
| Example 3 | 32.0 |

Test Example 2

The EdU positive rate of the intestinal stem cells obtained in the middle of Comparative Examples 1 and 1 to 3 was evaluated. After culturing for 24 hours after the addition of EdU, the intestinal stem cells obtained in Comparative Example 1 (on the 12th day) and Examples 1 to 3 (on the 14th day) were washed with D-PBS, and immersed in ice-cold methanol to be subjected to fixation and membrane permeation treatment. Then, the staining was carried out according to the manual attached to a Click-iT EdU Imaging Kits (Thermo Fisher Scientific, Inc.), and the observation was carried out with a fluorescence microscope. The EdU positive rate was calculated by dividing the number of nuclei stained with the Click-iT EdU Imaging Kits by the number of nuclei stained with Hoechst 33342. The results are shown in Table 2. As compared with Comparative Example 1, Example 1 showed a high EdU positive rate.

[Table 2]

|  | EdU positive rate (%) |
| --- | --- |
| Comparative Example 1 | 27 |
| Example 1 | 100 |

Test Example 3

The cell density of the intestinal epithelial cells obtained in Comparative Examples 1 and Examples 1 to 3 were evaluated. After the end of the induction of differentiation, the cells were washed with D-PBS, immersed in ice-cold methanol, and subjected to fixation and membrane permeation treatment. Then, DAPI was added thereto, followed by staining at room temperature for 5 minutes and replacement with D-PBS, and then observation was carried out with a fluorescence microscope. The cell density was calculated by counting the number of nuclei stained with DAPI. The results are shown in Table 3. As compared with Comparative Example 1, Examples 1 to 3 showed a high density of intestinal epithelial cells.

[Table 3]

|  | Cell density at final day of differentiation [$\times 10^4$ cells/cm$^2$] |
| --- | --- |
| Comparative Example 1 | 21.7 |
| Example 1 | 74.6 |
| Example 2 | 78.7 |
| Example 3 | 80.1 |

Test Example 4

The cell morphology of the intestinal epithelial cells obtained in Comparative Examples 1 and Examples 1 to 5 were evaluated. After the end of the induction of differentiation, the cells were fixed with a 2.5% glutaraldehyde phosphate buffer solution. The membrane to which the cells adhered was cut out from the cell culture insert and embedded in paraffin. Sections were sliced at a section thickness of 2 to 3 $\mu$m and stained with hematoxylin 3G and eosin. The observation was carried out after using a mounting agent (Paramount, Pharma). A cell in which the longest axis of the cell was 3 times or more with respect to the longest axis of the nucleus was regarded as a column-shaped cell, and the proportion of the column-shaped cell to the entire section was evaluated. The results are shown in Table 4. In Comparative Example 1, column-shaped cells accounted for 0%, whereas in Examples 1 to 5, column-shaped cells accounted for 20% to 100%.

[Table 4]

|  | Proportion of column-shaped cell (%) |
| --- | --- |
| Comparative Example 1 | 0 |
| Example 1 | 100 |
| Example 2 | 20 |
| Example 3 | 100 |
| Example 4 | 100 |
| Example 5 | 80 |

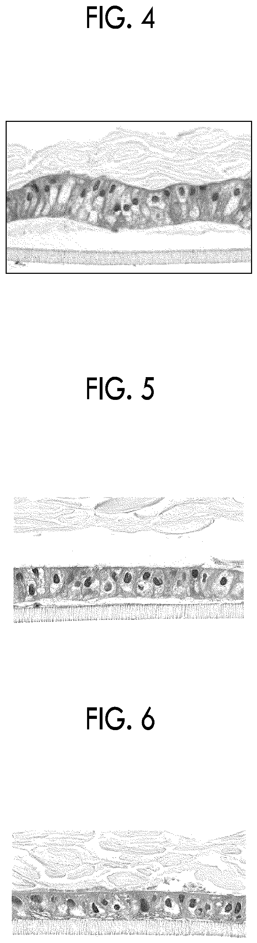

In addition, images acquired by slide scanning of sliced sections are shown in Fig. 2 (Comparative Example 1), Fig. 3 (Example 1), Fig. 4 (Example 3), Fig. 5 (Example 4), and Fig. 6 (Example 5). It was found that Examples 1, 3, 4, and 5 show a column-shaped cell morphology as compared with Comparative Example 1, and the morphology is closer to that of the small intestine of a living body.

Test Example 5

The CYP3A4 activity per area of the intestinal epithelial cell sheet obtained in Comparative Example 1 and Examples 1 to 5 was measured from the amount of a metabolite of midazolam. After the end of the induction of differentiation, the cell sheet was incubated in a culture medium containing 5 μmol/L midazolam at 37°C, and after 2 hours, the culture medium was sampled. The metabolic activity was calculated from the amount of 1'-hydroxymidazolam in the culture medium, which was measured using a liquid chromatography-mass spectrometer (LC-MS/MS). The results are shown in Table 5. As compared with Comparative Example 1, Examples 1 to 5 showed a high amount of the midazolam metabolite per area of the cell sheet.

[Table 5]

|  | Amount of midazolam metabolite per area (pmol/2 hr/cm$^2$) | CYP3A4 activity per protein mass (pmol/2 hr/cm$^2$) |
|---|---|---|
| Comparative Example 1 | 213 | 638 |
| Example 1 | 432 | 530 |
| Example 2 | 681 | 1487 |
| Example 3 | 630 | 1298 |
| Example 4 | 423 | 494 |
| Example 5 | 426 | 678 |

Test Example 6

The midazolam permeability of the intestinal epithelial cells obtained in Comparative Examples 1 and Examples 1 to 5 was evaluated. After the end of the induction of differentiation, HBSS (pH 6.5) was added to the apical side and HBSS (pH 7.4) was added to the lower bottom (basolateral) side, and pre-incubation was carried out at 37°C for 60 minutes. Then, HBSS (pH 6.5) containing 10 μmol/L midazolam was added to the apical side, and HBSS (pH 7.4) was added to the lower bottom (basolateral) side, and incubation was carried out at 37°C for 60 minutes. A sample was collected from the lower bottom (basolateral) side every 15 minutes. The amount of each compound (unchanged form) was quantified using a liquid chromatography-mass spectrometer (LC-MS/MS). The apparent permeability coefficient (Papp) was calculated from the quantification result. The results are shown in Table 6. Since Examples 1 to 5 showed a decrease in apparent permeability coefficient (Papp) as compared with Comparative Example 1, it can be said that the accuracy of the kinetic prediction is improved.

[Table 6]

|  | Apparent permeability coefficient [× 10$^{-6}$ cm/s] |
|---|---|
| Comparative Example 1 | 48 |
| Example 1 | 30 |
| Example 2 | 32 |
| Example 3 | 33 |
| Example 4 | 31 |
| Example 5 | 31 |

Test Example 7

The apparent permeability coefficient was corrected by using the scaling factor x for the apparent permeability coefficient of Example 4 obtained in Test Example 6. At this time, the following expression was used.
The permeation amount per unit time = (the B60 substrate amount - x × the difference in the B60 substrate amount due to the presence or absence of the addition of the metabolism inhibitor)/the permeation time
By using the scaling factor, the apparent permeability coefficient obtained in a case of using the small intestine cell sheet according to the embodiment of the present invention was closer to a value close to the apparent permeability coefficient obtained in a case of using the small intestinal tissue of a living body. As a result, the accuracy of the kinetic prediction was improved.

[Table 7]

| X | 2 | 3 | 4 | 5 | 5.2 |
|---|---|---|---|---|---|
| Apparent permeability coefficient after correction [$\times 10^{-6}$ cm/s] | 21.9 | 15.2 | 8.6 | 1.9 | 0.6 |

Test Example 8

In Example 4, it was found that Fg simply calculated by substituting the experimental numerical values obtained as a result of carrying out Test Example 6 into Expression 1 is 0.83.

$$Fg = 431/(431 + 91) = 0.83$$

As a result of correcting Fg using the scaling factor x in Expression 2, the obtained correction results are as shown in the following table.

$$Fg = 431/(431 + 91 \times x) \cdots (\text{Expression 2})$$

As a result of correcting Fg with x = 4 to 12, the apparent Fg of midazolam was 0.28 to 0.54, and an Fg equivalent to 0.369 to 0.5, which is the Fg of the small intestine of a living body, was calculated, and thus the prediction accuracy was improved.

[Table 8]

| X | 1 | 2 | 4 | 6 | 8 | 10 | 12 |
|---|---|---|---|---|---|---|---|
| Fg after correction | 0.83 | 0.70 | 0.54 | 0.44 | 0.37 | 0.32 | 0.28 |

**Claims**

1. A production method for an intestinal epithelial cell, comprising:

    a first differentiation step of differentiating a pluripotent stem cell into an intestinal stem cell;
    a proliferation step of proliferating the intestinal stem cell obtained in the differentiation step; and
    a second differentiation step of differentiating the intestinal stem cell obtained in the proliferation step into an intestinal epithelial cell,
    wherein the proliferation step is any one of;
    a step in which a density of the intestinal stem cells obtained in the first differentiation step becomes $30 \times 10^4$ cells/cm$^2$ or more,
    a step in which the density of the intestinal stem cells obtained in the first differentiation step becomes 2.0 times or more as compared with a density of the intestinal stem cells immediately after the first differentiation step,
    a step in which an EdU-positive proportion of the intestinal stem cells obtained in the first differentiation step becomes 90% or more, or
    a step in which the EdU-positive proportion of the intestinal stem cells obtained in the first differentiation step becomes 3 times or more as compared with a proportion of the intestinal stem cells immediately after the first differentiation step.

2. The production method for an intestinal epithelial cell according to claim 1,
    wherein the proliferation step includes a step of culturing the intestinal stem cell in a presence of a Wnt agonist or a GSK-3β inhibitor.

3. The production method for an intestinal epithelial cell according to claim 1,
    wherein the proliferation step includes a step of culturing the intestinal stem cell in a presence of a GSK-3β inhibitor and a serum substitute.

4. The production method for an intestinal epithelial cell according to any one of claims 1 to 3,
   wherein a cell density at an end of the first differentiation step is $5.0 \times 10^4$ cells/cm$^2$ to $20 \times 10^4$ cells/cm$^2$.

5. The production method for an intestinal epithelial cell according to any one of claims 1 to 4,
   wherein in the proliferation step, the intestinal stem cells obtained in the first differentiation step are seeded in a container to a cell density of 1.1 to 3.3 times, and then the intestinal stem cells are cultured and proliferated.

6. The production method for an intestinal epithelial cell according to any one of claims 1 to 5,
   wherein the proliferation step is carried out for 1 to 10 days.

7. The production method for an intestinal epithelial cell according to any one of claims 1 to 6,
   wherein the proliferation step and the second differentiation step are carried out in a culture container coated with an extracellular matrix of 3% by volume to 100% by volume.

8. The production method for an intestinal epithelial cell according to any one of claims 1 to 6,
   wherein the proliferation step and the second differentiation step are carried out in a culture medium to which an extracellular matrix of 1% to 10% is added.

9. The production method for an intestinal epithelial cell according to claim 8,
   wherein the proliferation step and the second differentiation step are carried out in a culture medium in which the extracellular matrix of 1% to 10% is added to only a basolateral side of the cell.

10. The production method for an intestinal epithelial cell according to any one of claims 1 to 9,
    wherein the second differentiation step includes a step of differentiating the intestinal stem cell into the intestinal epithelial cell in a presence of a MEK1 inhibitor, a DNA methylation inhibitor, a TGFβ receptor inhibitor, EGF, and a cAMP activator.

11. An intestinal epithelial cell that is produced by the method according to any one of claims 1 to 10.

12. A cell sheet comprising the intestinal epithelial cell according to claim 11.

13. A cell sheet comprising:

    an intestinal epithelial cell derived from a pluripotent stem cell,
    wherein a proportion of columnar intestinal epithelial cells in the intestinal epithelial cells is 10% or more.

14. The cell sheet according to claim 13,
    wherein a cell density is $50 \times 10^4$ cells/cm$^2$ or more.

15. The cell sheet according to claim 13 or 14,
    wherein an amount of a midazolam metabolite per area after incubation in a culture medium containing 5 μmol/L midazolam at 37°C for 2 hours is 300 pmol/cm$^2$ or more per area of the cell sheet.

16. The cell sheet according to any one of claims 13 to 15,
    wherein a CYP3A4 activity per protein mass is 400 to 2,500 pmol/2 hr/mg·protein.

17. The cell sheet according to any one of claims 13 to 16,
    wherein an apparent permeability coefficient of midazolam is $40 \times 10^{-6}$ cm/s or less.

18. A evaluation method for a test substance, comprising bringing a test substance into contact with the intestinal epithelial cell according to claim 11 or the cell sheet according to any one of claims 12 to 17.

19. The evaluation method for a test substance according to claim 18, further comprising correcting an apparent permeability coefficient using a scaling factor.

20. The evaluation method for a test substance according to claim 18, further comprising correcting Fg using a scaling factor.

21. The evaluation method for a test substance according to claim 19 or 20,
    wherein the scaling factor is 0.1 to 500.

22. A screening kit for a test substance, comprising the intestinal epithelial cell according to claim 11 or the cell sheet according to any one of claims 12 to 17.

23. A cell preparation comprising the intestinal epithelial cell according to claim 11 or the cell sheet according to any one of claims 12 to 17.

# FIG. 1

COMPARATIVE EXAMPLE 1

> ENDODERM >> INTESTINAL STEM CELL >> INTESTINAL EPITHELIAL CELL >

EXAMPLES 1 TO 5

PROLIFERATION OF INTESTINAL STEM CELL

> ENDODERM >> INTESTINAL STEM CELL >> INTESTINAL EPITHELIAL CELL >

# FIG. 2

# FIG. 3

## FIG. 4

## FIG. 5

## FIG. 6

## INTERNATIONAL SEARCH REPORT

| | International application No. |
|---|---|
| | PCT/JP2021/023893 |

### A. CLASSIFICATION OF SUBJECT MATTER

C12N 5/071(2010.01)i; A61K 35/38(2015.01)i; A61L 27/38(2006.01)i; A61P 1/00(2006.01)i; C12Q 1/02(2006.01)i; G01N 33/15(2006.01)i; G01N 33/50(2006.01)i; C12N 5/10(2006.01)n
FI:    C12N5/071; A61K35/38; A61L27/38 300; A61P1/00; C12Q1/02; G01N33/15 Z; G01N33/50 Z; C12N5/10

According to International Patent Classification (IPC) or to both national classification and IPC

### B. FIELDS SEARCHED

Minimum documentation searched (classification system followed by classification symbols)
C12N5/071; A61K35/38; A61L27/38; A61P1/00; C12Q1/02; G01N33/15; G01N33/50; C12N5/10

Documentation searched other than minimum documentation to the extent that such documents are included in the fields searched
| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN); Japio-GPG/FX

### C. DOCUMENTS CONSIDERED TO BE RELEVANT

| Category* | Citation of document, with indication, where appropriate, of the relevant passages | Relevant to claim No. |
|---|---|---|
| X | WO 2018/151307 A1 (NAGOYA CITY UNIVERSITY) 23 August 2018 (2018-08-23) claims, examples | 1-23 |
| X | WO 2019/156200 A1 (NAGOYA CITY UNIVERSITY) 15 August 2019 (2019-08-15) claims, examples 1-2 | 11-12, 18-23 |
| A | | 1-10, 13-17 |
| A | WO 2014/132933 A1 (NAGOYA CITY UNIVERSITY) 04 September 2014 (2014-09-04) entire text, all drawings | 1-23 |

☐ Further documents are listed in the continuation of Box C.　　☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 August 2021 (16.08.2021) | 24 August 2021 (24.08.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2021/023893

| Patent Documents referred in the Report | Publication Date | Patent Family | Publication Date |
|---|---|---|---|
| WO 2018/151307 A1 | 23 Aug. 2018 | US 2020/0002680 A1 claims, examples EP 3584313 A1 | |
| WO 2019/156200 A1 | 15 Aug. 2019 | (Family: none) | |
| WO 2014/132933 A1 | 04 Sep. 2014 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018151307 A
- WO 2019156200 A
- WO 2014165663 A

### Non-patent literature cited in the description

- Manipulating the Mouse Embryo A Laboratory Manual. Cold Spring Harbor Laboratory Press
- THOMSON, J. A. *Science,* 1998, vol. 282, 1145-1147
- WILMUT et al. *Nature,* 1997, vol. 385, 810
- CIBELLI et al. *Science,* 1998, vol. 280, 1256
- IRIYA et al. *Protein Nucleic Acid Enzyme,* 1999, vol. 44, 892
- BAGUISI et al. *Nature Biotechnology,* 1999, vol. 17, 456
- WAKAYAMA et al. *Nature,* 1998, vol. 394, 369
- *Nature Genetics,* 1999, vol. 22, 127
- *Proc. Natl. Acad. Sci. USA,* 1999, vol. 96, 14984
- RIDEOUT III et al. *Nature Genetics,* 2000, vol. 24, 109
- TACHIBANA et al. Human Embryonic Stem Cells Derived by Somatic Cell Nuclear Transfer. *Cell,* 2013
- KIM et al. *Science,* 2007, vol. 315, 482-486
- NAKAJIMA et al. *Stem Cells,* 2007, vol. 25, 983-985
- KIM et al. *Cell Stem Cell,* 2007, vol. 1, 346-352
- REVAZOVA et al. *Cloning Stem Cells,* 2007, vol. 9, 432-449
- REVAZOVA et al. *Cloning Stem Cells,* 2008, vol. 10, 11-24
- STRELCHENKO N. et al. *Reprod Biomed Online,* 2004, vol. 9, 623-629
- KLIMANSKAYA I. et al. *Nature,* 2006, vol. 444, 481-485
- CHUNG Y. et al. *Cell Stem Cell,* 2008, vol. 2, 113-117
- ZHANG X. et al. *Stem Cells,* 2006, vol. 24, 2669-2676
- WASSARMAN, P. M. et al. *Methods in Enzymology,* 2003, vol. 365
- MATSUI et al. *Cell,* 1992, vol. 70, 841-847
- SHAMBLOTT et al. *Proc. Natl. Acad. Sci. USA,* 1998, vol. 9523, 13726-13731
- TURNPENNY et al. *Stem Cells,* 2003, vol. 21 (5), 598-609
- TAKAHASHI K ; YAMANAKA S. *Cell,* 2006, vol. 126 (4), 663-676
- TAKAHASHI, K et al. *Cell,* 2007, vol. 131 (5), 861-72
- YU J et al. *Science,* 2007, vol. 318 (5858), 1917-1920
- NAKAGAWA M et al. *Nat. Biotechnol.,* 2008, vol. 26 (1), 101-106
- KIM J B et al. *Nature,* 2008, vol. 454 (7204), 646-650
- KIM J B et al. *Cell,* 2009, vol. 136 (3), 411-419
- ZHOU H ; WU S ; JOO J Y et al. *Cell Stem Cell,* 2009, vol. 4, 381-384
- KIM D ; KIM C H ; MOON J I et al. *Cell Stem Cell,* 2009, vol. 4, 472-476
- HUANGFU D et al. *Nat. Biotechnol.,* 2008, vol. 26 (7), 795-797
- HUANGFU D et al. *Nat. Biotechnol.,* 2008, vol. 26 (11), 1269-1275
- SILVA J et al. *PLoS. Biol.,* 2008, vol. 6 (10), e253
- STADTFELD M et al. *Science,* 2008, vol. 322 (5903), 945-949
- OKITA K et al. *Science,* 2008, vol. 322 (5903), 949-953
- WOLTJEN K ; MICHAEL I P ; MOHSENI P et al. *Nature,* 2009, vol. 458, 766-770
- KAJI K ; NORRBY K ; PACA A et al. *Nature,* 2009, vol. 458, 771-775
- YUSA K ; RAD R ; TAKEDA J et al. *Nat Methods,* 2009, vol. 6, 363-369
- YU J ; HU K ; SMUGA-OTTO K ; TIAN S et al. *Science,* 2009, vol. 324, 797-801
- *CHEMICAL ABSTRACTS,* 76939-46-3
- *CHEMICAL ABSTRACTS,* 1135306-29-4
- *CHEMICAL ABSTRACTS,* 151837-09-1
- *CHEMICAL ABSTRACTS,* 93602-66-5
- *CHEMICAL ABSTRACTS,* 16980-89-5
- *CHEMICAL ABSTRACTS,* 124705-03-9
- *CHEMICAL ABSTRACTS,* 129735-00-8
- *CHEMICAL ABSTRACTS,* 93882-12-3
- *CHEMICAL ABSTRACTS,* 634207-53-7

- **A-L. UNGEL et al.** *Eur. J. Phrm. Sci.,* 2013, vol. 48, 166
- **TAKENAKA T. et al.** *Drug Metab Dispos,* 2014, vol. 42, 1947, , 54
- *Pharm Res,* March 2012, vol. 29 (3), 651
- **TAKAYAMA K. et al.** *Cell. Mol. Gastroenterol. Hepatol.,* 2019, vol. 8, 513-526